# EUROPEAN PATENT APPLICATION

(11) **EP 4 180 035 A1**
(43) Date of publication of application: **17.05.2023**
(21) Application number: 21208202.8
(22) Date of filing: 15.11.2021
(51) Int. Cl.: A61K 31/397, A01N 31/00, A01P 1/00, A61P 29/00, A61P 33/00, A61P 35/00, A61P 37/00

(54) **NOVEL BETA-LACTONE INHIBITORS OF HYDROLYTIC ENZYMES AND THEIR MEDICAL AND NON MEDICAL USES**

(71) Applicant: Max-Planck-Gesellschaft zur Förderung der Wissenschaften e.V., 80539 München (DE); Technische Universität München, 80333 München (DE); Johann-Wolfgang-Goethe-Universität Frankfurt am Main, 60323 Frankfurt am Main (DE)
(72) Inventor: BODE, Helge B., 61440 Oberursel (DE); SHI, Yi-Ming, 60438 Frankfurt am Main (DE); GROLL, Michael, 80333 München (DE); KUTTENLOCHNER, Wolfgang, 80333 München (DE)
(74) Representative: Vossius & Partner Patentanwälte Rechtsanwälte mbB

(57) **Abstract**

The present invention relates to novel beta-lactone inhibitors of hydrolytic enzymes and their medical use, in particular a compound of formula (I) or a pharmaceutically acceptable salt thereof, for use in therapy. The present invention further relates to a pharmaceutical composition comprising the compound of formula (I) as defined herein and a pharmaceutically acceptable excipient. The instant compounds and compositions are useful in the treatment or prevention of cancer, an infectious disease, an inflammatory disease, or an autoimmune disease, and are useful as proteasome inhibitors. The present invention further relates to a plant protection composition comprising a compound of formula (I) or an agriculturally acceptable salt thereof, and to a method of controlling a plant disease, the method comprising applying a compound or composition according to the invention. The present invention further relates to a disinfectant comprising the compound of formula (I) and to the non-therapeutic use of the instant compounds or disinfectants for disinfecting or sterilizing an inanimate object.

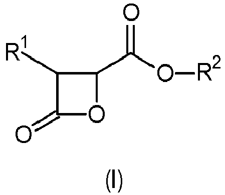

## Description

The present invention relates to novel beta-lactone inhibitors of hydrolytic enzymes and their medical use, in particular a compound of formula (I) or a pharmaceutically acceptable salt thereof, for use in therapy. The present invention further relates to a pharmaceutical composition comprising the compound of formula (I) as defined herein and a pharmaceutically acceptable excipient. The instant compounds and compositions are useful in the treatment or prevention of cancer, an infectious disease, an inflammatory disease, or an autoimmune disease, and are useful as proteasome inhibitors. The present invention further relates to a plant protection composition comprising a compound of formula (I) or an agriculturally acceptable salt thereof, and to a method of controlling a plant disease, the method comprising applying a compound or composition according to the invention. The present invention further relates to a disinfectant comprising the compound of formula (I) and to the non-therapeutic use of the instant compounds or disinfectants for disinfecting or sterilizing an inanimate object.

The 20S proteasome is a valuable target for the treatment of a number of diseases including cancer, neurodegenerative diseases, and parasitic infections. The ubiquitin-proteasome pathway plays a role in regulating cell cycle, neoplastic growth and metastasis. A number of key regulatory proteins, including cyclins, and the cyclin-dependent kinases p21 and p27^{KSF1}, are temporally degraded during the cell cycle by the ubiquitin-proteasome pathway. The ordered degradation of these proteins is required for the cell to progress through the cell cycle and to undergo mitosis. Furthermore, the ubiquitin-proteasome pathway is required for transcriptional regulation. The ubiquitin-proteasome pathway is required for expression of cell adhesion molecules, such as E-selectin, ICAM-I, and VCAM-I. The cell adhesion molecules are involved in tumor metastasis and angiogenesis in vivo, by directing the adhesion and extravastation of tumor cells to and from the vasculature to distant tissue sites within the body.

In addition to the commonly expressed constitutive proteasome core particle (CP), there is an alternative complex immunoproteasome that can be found in immune cells and/or cells exposed to inflammatory cytokines such as IFN-γ and TNF-α. The immunoproteasome differs from the constitutive proteasome in its subunit composition. It contains subunits with chymotrypsin-like (β5i / LMP7), caspase-like (β1i / LMP2), and trypsin-like (β2i) protease activity, which replace the corresponding counterparts in the constitutive proteasome (β5c, β1c, and β2c, respectively). When all three IFN-γ-inducible subunits are present, the proteasome is called an "immunoproteasome". The core particle of the immunoproteasome, which may also be referred to as iCP, efficiently generates oligopeptides with hydrophobic C-terminal anchor residues and a high affinity for the MHC-I receptor, thereby supporting antigen presentation as well as pathogen clearance. This physiological function of the iCP is based on its altered substrate cleavage preferences emanating from the subunits β1i, β2i and β5i. Selective inhibition of the iCP could be beneficial for the treatment of autoimmune diseases.

Only in recent years, two additional CP types have been discovered, i.e., the thymoproteasome core particle (tCP) and the spermatoproteasome core particle (sCP). The thymoproteasome core particle (tCP), exclusively present in cortical epithelial cells of the thymus, drives maturation and positive selection of T cells by producing low affinity peptides for MHC-I receptors. This process is crucial for establishing self-tolerance as well as preventing autoimmunity and relies on the unique subunit composition of the tCP (β1i, β2i and β5t). The spermatoproteasome core particle (sCP) is required for proper spermatogenesis in mammalian testes and incorporates a variant of subunit a4, thereby altering the interaction with regulatory particles.

The proteasome inhibitor VELCADE^{®} (bortezomib; N-2-pyrazinecarbonyl-L-phenylalanine-L-leucineboronic acid) is the first proteasome inhibitor to achieve regulatory approval. Bortezomib was approved for the treatment of multiple myeloma patients who have received at least one prior therapy. Further studies confirmed the activity of bortezomib in patients with relapsed or refractory mantle cell lymphoma. Besides bortezomib (Velcade^{®}), the proteasome inhibitors carfilzomib (Kyprolis^{®}) and ixazomib (Ninlaro^{®}) have also been found to significantly improve the clinical outcome of multiple myeloma patients by non-selectively targeting CPs and killing cancer cells that strictly rely on high-capacity protein degradation.

Certain lactones and other compounds have been proposed as proteasome inhibitors in the literature (Huber EM et al., A Nut for Every Bolt: Subunit-Selective Inhibitors of the Immunoproteasome and Their Therapeutic Potential, Cells, 10(8), 1929, (2021); Groll M et al., (-)-Homosalinosporamide A and its mode of proteasome inhibition: an X-ray crystallographic study, Mar. Drugs, 16(7), 240, (2018); Groll M et al., Ein minimales β-Lacton-Gerüst für selektive β5c- oder β5i-Proteasominhibitoren, Angewandte Chemie, 127, 7921-7925 (2015); List A et al., Omuralide and Vibralactone: Differences in the Proteasome-β-Lactone-γ-Lactam Binding Scaffold Alter Target Preferences, Angewandte Chemie International Edition, 53, 571-574 (2014); Kawamura S et al., Potent Proteasome Inhibitors Derived from the Unnatural cis-Cyclopropane Isomer of Belactosin A: Synthesis, Biological Activity, and Mode of Action, J. Med. Chem. 56, 9, 3689-3700 (2013); Huber EM et al., Inhibitors for the immuno- and constitutive proteasome: current and future trends in drug development, Angewandte Chemie International Edition, 51(35), 8708-20, (2012); Korotkov VS et al., Synthesis and biological activity of optimized belactosin C congeners, Org. Biomol. Chem., 9, 7791-7798 (2011); Groll M et al., Structural Analysis of Spiro β-Lactone Proteasome Inhibitors, J. Med. Chem. 52, 17, 5420-5428 (2009); Manam RR et al., Leaving Groups Prolong the Duration of 20S Proteasome Inhibition and Enhance the Potency of Salinosporamides, J. Med. Chem. 51, 21, 6711-6724 (2008); Groll M et al., Snapshots of the Fluorosalinosporamide/20S Complex Offer Mechanistic Insights for Fine Tuning Proteasome Inhibition, J. Am. Chem. Soc., 130, 45, 14981-14983, (2008); WO 2019/213386; WO 2016/123699; WO 2009/140287; WO 2008/006113).

As the proteasome represents an important target for therapeutic intervention, there is a continued need for new and/or improved proteasome inhibitors.

It is thus an object of the present invention to provide novel and/or improved inhibitors of hydrolytic enzymes, in particular novel and/or improved proteasome inhibitors.

In the context of the present invention it was surprisingly found that the compounds of general formula (I) as described hereinbelow are highly potent inhibitors of the eukaryotic proteasome, and therefore are particulary well suited for therapeutic use, e.g. for the treatment or prevention of cancer, an infectious disease, an inflammatory disease, or an autoimmune disease.

Intense research efforts have also been devoted to the characterization of unknown biosynthetic gene clusters (BGCs) for natural product discovery, as well as functional assignments of natural products in the context of bacteria-nematode-insect interactions. The present inventors have identified a previously unknown biosynthetic gene cluster (also referred to as BGC), referred to as *ioclleu* BGC, which is proposed to be related to β-lactone biosynthesis.

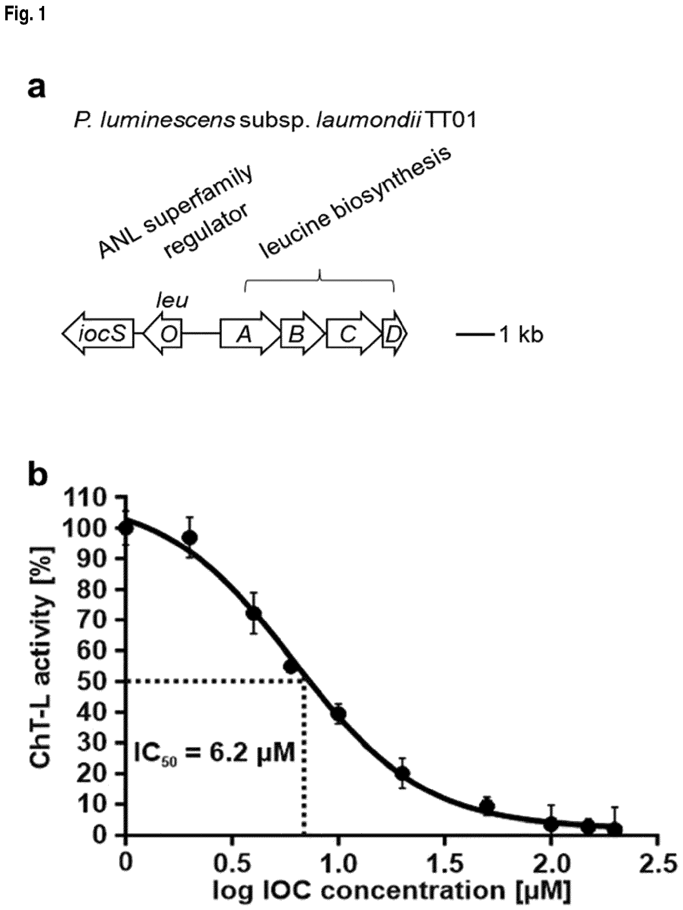

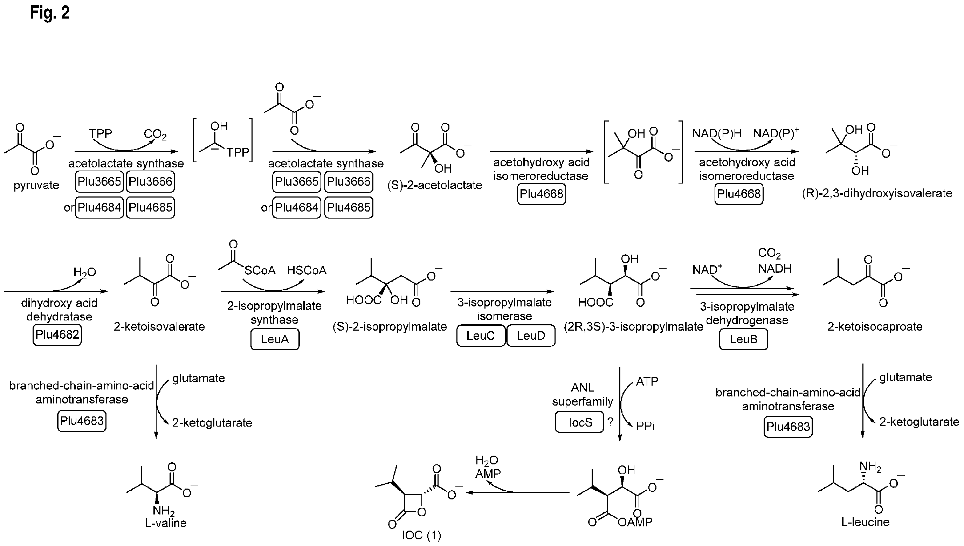

Recognizing that the putative β-lactone producing BGC is highly expressed under normal laboratory conditions, the present inventors have attempted to predict a possible chemical structure based on the functions of biosynthetic genes of *ioclleu* BGC. The putative biosynthetic cluster features six genes (**Fig. 1a**). *leuABCD* are involved in the L-leucine biosynthesis. *leuO* is positioned next to *leuA* and encodes a global transcription factor involved in regulating natural product biosynthesis (Engel, Y., Windhorst, C., Lu, X., Goodrich-Blair, H. & Bode, H. B. The Global Regulators Lrp, LeuO, and HexA Control Secondary Metabolism in Entomopathogenic Bacteria. Frontiers in microbiology 8, 209 (2017)) and other physiological traits (Whitaker, W. B., Parent, M. A., Boyd, A., Richards, G. P. & Boyd, E. F. The Vibrio parahaemolyticus ToxRS regulator is required for stress tolerance and colonization in a novel orogastric streptomycin-induced adult murine model. Infect. Immun. 80, 1834-1845 (2012)). *iocS* encodes an enzyme belonging to the ANL (acyl-CoA synthetases, NRPS adenylation domains, and luciferase enzymes) superfamily. Such a gene architecture is reminiscent of the biosynthesis of cystargolides (Wolf, F. et al. Biosynthesis of the beta-Lactone Proteasome Inhibitors Belactosin and Cystargolide. Angew. Chem. Int. Ed. Engl. 56, 6665-6668 (2017)), during which 3-isopropylmalate as an intermediate in the leucine pathway is the precursor for one-step lactonization to afford 3-isopropyl-4-oxo-2-oxetanecarboxylic acid ("IOC" or compound **1**) with a β-lactone moiety (**Fig. 2**). Although the enzyme responsible for β-lactonization remains uncharacterized in the cystargolide biosynthesis, a recent report demonstrates the acyl-AMP ligase, OleC, to be a β-lactone synthetase during the biosynthesis of long-chain olefinic hydrocarbons (Robinson, S. L., Christenson, J. K. & Wackett, L. P. Biosynthesis and chemical diversity of beta-lactone natural products. Nat. Prod. Rep. 36, 458-475 (2018)). Therefore, it has been proposed that locS might be responsible for adenylating the 4-carboxyl group and then triggering lactonization to give compound **1**.

The *ioclleu* BGC responsible for compound **1** biosynthesis was surprisingly found by the present inventors to be present across all genomes of γ-proteobacteria *Xenorhabdus* and *Photorhabdus* (*XP*)*.* The *ioclleu* BGC is also widely distributed in other γ-Proteobacteria like free-living pathogens *Vibrio cholerae* and *Yersinia pestis.* Although this BGC has yet to be studied in other microorganisms and the degree of structural conservation of the compound **1** among γ-Proteobacteria is unknown, it is conceivable that the conservation of structural genes, *leuA-D* for L-leucine biosynthesis and *iocS* for putative lactonization, can serve as an indicator that compound **1** is highly conserved among γ-Proteobacteria targeting the eukaryotic proteasome.

In a first embodiment, the present invention thus relates to a compound of formula (I): 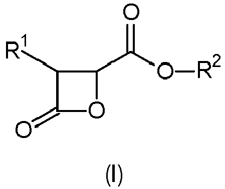 or a pharmaceutically acceptable salt thereof, for use in therapy.

In formula (I), R¹ is selected from hydrogen, C₁₋₈ alkyl, C₂₋₈ alkenyl, C₂₋₈ alkynyl, -(C₁₋₆ alkylene)-O-R¹¹, -(C₁₋₆ alkylene)-S-R¹¹, -(C₁₋₆ alkylene)-N(R¹¹)-R¹¹, -(C₁₋₆ alkylene)-CO-R¹¹, -(C₁₋₆ alkylene)-COO-R¹¹, -(C₁₋₆ alkylene)-O-CO-(C₁₋₆ alkyl), -(C₁₋₆ alkylene)-CO-N(R¹¹)-R¹¹, -(C₁₋₆ alkylene)-N(R¹¹)-CO-(C₁₋₆ alkyl), -(C₁₋₆ alkylene)-CO-N(R¹¹)-O-R¹¹, -(C₁₋₆ alkylene)-O-CO-N(R¹¹)-R¹¹, -(C₁₋₆ alkylene)-N(R¹¹)-CO-N(R¹¹)-R¹¹, -(C₁₋₆ alkylene)-N(R¹¹)-C(=N-R¹¹)-N(R¹¹)-R¹¹, - (C₁₋₆ alkylene)-SO₃-R¹¹, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R³, wherein said alkyl, said alkenyl, said alkynyl, and any alkylene group comprised in any of the aforementioned R¹ groups are each optionally substituted with one or more -OH, and further wherein each R¹¹ is independently selected from hydrogen and C₁₋₆ alkyl.

R² is selected from hydrogen, C₁₋₆ alkyl, C₂₋₆ alkenyl, and C₂₋₆ alkynyl.

Each R³ is independently selected from C₁₋₆ alkyl, C₂₋₆ alkenyl, C₂₋₆ alkynyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -S(C₁₋₆ alkylene)-SH, -S(C₁₋₆ alkylene)-S(C₁₋₆ alkyl), -(C₁₋₆ alkylene)-SH, -(C₁₋₆ alkylene)-S(C₁₋₆ alkyl), -NH₂, -NH(C₁₋₆ alkyl), -N(C₁₋₆ alkyl)(C₁₋₆ alkyl), -NH-OH, -N(C₁₋₆ alkyl)-OH, -NH-O(C₁₋₆ alkyl), -N(C₁₋₆ alkyl)-O(C₁₋₆ alkyl), =NH, =N(C₁₋₆ alkyl), halogen, C₁₋₆ haloalkyl, -O-(C₁₋₆ haloalkyl), -CN, -NO₂, -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), -SO-(C₁₋₆ alkyl), -(C₀₋₄ alkylene)-carbocyclyl, and -(C₀₋₄ alkylene)-heterocyclyl, wherein the carbocyclyl group in said -(C₀₋₄ alkylene)-carbocyclyl and the heterocyclyl group in said -(C₀₋₄ alkylene)-heterocyclyl are each optionally substituted with one or more groups independently selected from C₁₋₆ alkyl, C₂₋₆ alkenyl, C₂₋₆ alkynyl, halogen, C₁₋₆ haloalkyl, -O-(C₁₋₆ haloalkyl), -CN, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), and -SO-(C₁₋₆ alkyl).

In a further embodiment, the present invention relates to a pharmaceutical composition comprising the compound of formula (I) or a pharmaceutically acceptable salt thereof and a pharmaceutically acceptable excipient. The invention likewise relates to the use of a compound of formula (I) or a pharmaceutically acceptable salt thereof in the manufacture of a medicament.

In a further embodiment, the present invention relates to the compound of formula (I) or the pharmaceutically acceptable salt thereof or the pharmaceutical composition of the present invention for use in the treatment or prevention of cancer, an infectious disease, an inflammatory disease, or an autoimmune disease.

In a further embodiment, the present invention relates to use of the compound of formula (I) or a pharmaceutically acceptable salt thereof in the manufacture of a medicament for treatment or prevention of cancer, an infectious disease, an inflammatory disease, or an autoimmune disease.

In a further embodiment, the present invention relates to a method of treating or preventing cancer, an infectious disease, an inflammatory disease, or an autoimmune disease, the method comprising administering a compound of formula (I) or a pharmaceutically acceptable salt thereof, or a pharmaceutical composition comprising any of the aforementioned entities in combination with a pharmaceutically acceptable excipient, to a subject (preferably a human) in need thereof. It will be understood that a therapeutically effective amount of the compound of formula (I) or the pharmaceutically acceptable salt thereof (or of the pharmaceutical composition) is to be administered in accordance with this method.

In a further embodiment, the present invention relates to a plant protection composition comprising a compound of formula (I) or an agriculturally acceptable salt thereof.

In a further embodiment, the present invention relates to a method of controlling a plant disease, the method comprising applying a compound of formula (I) or an agriculturally acceptable salt thereof or the plant protection composition of the present invention. It will be understood that an effective amount of the compound of formula (I) or the agriculturally acceptable salt thereof or the plant protection composition of the present invention should be applied, e.g., to the plant itself (including any specific part of the plant, such as its leaves, foliage, branches, or roots) or to the soil (where the plant is cultivated).

In a further embodiment, the present invention relates to a disinfectant comprising the compound of formula (I) or a salt thereof.

In a further embodiment, the present invention relates to the non-therapeutic use of the compound of formula (I) or a salt thereof or the disinfectant of the present invention for disinfecting or sterilizing an inanimate object.

In a further embodiment, the present invention relates to a method of disinfecting or sterilizing an inanimate object, the method comprising applying a compound of formula (I) or a salt thereof, or the disinfectant of the present invention as defined herein, to said inanimate object.

In a further embodiment, the present invention relates to the *in vitro* use of a compound of formula (I) or a pharmaceutically acceptable salt thereof of the present invention as a proteasome inhibitor.

The present invention is illustrated by the appended figures.

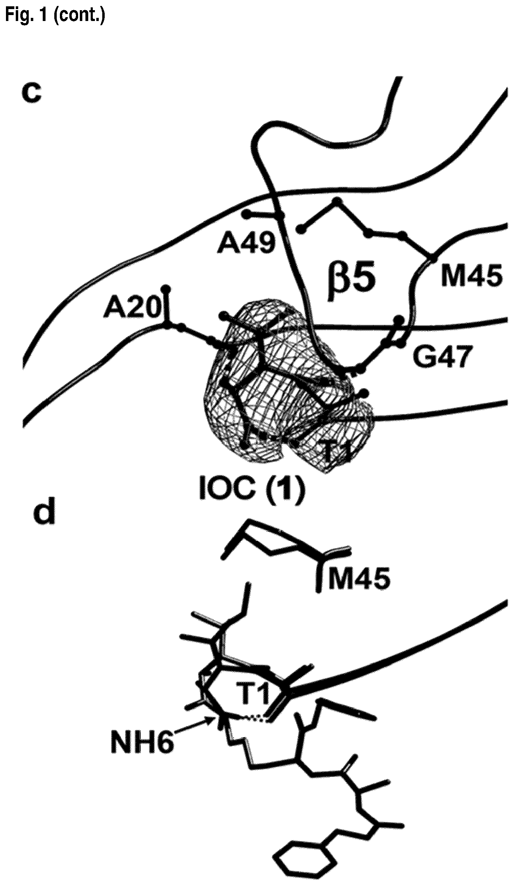

- **Fig. 1**: presents BGCs, chemical structures, and bioactivities of compound **1.** (**a**) Genetic architecture of the *ioc*/*leu* BGC. (**b**) Dose-response curve for IOC-mediated inhibition of the β5 subunit of the yeast 20S proteasome. The IC₅₀ value of compound **1** was determined to 6.2 ± 1.2 µM. Experiments were performed in triplicate. (**c**) Crystal structure of the yeast 20S proteasome in complex with compound **1** (spherical model) bound to the chymotrypsin-like active site (β5 subunit, PDB ID 7O2L); illustration of the 2*F*_{O}-*F*_{C} electron density map (grey mesh, contoured to 1σ) of compound **1** covalently linked through an ester bond to Thr1O^{γ} of subunit β5. Protein residues interacting with compound **1** are highlighted. Dots illustrate hydrogen bonds between compound **1** and protein residues. (**d**) Superposition of compound **1** and homobelactosin C (PDB ID 3E47) complex structures with the yeast 20S proteasome highlights similar conformations at the chymotrypsin-like active site.
- **Fig. 2**: presents a proposed biosynthetic pathway of compound **1** in *Photorhabdus luminescens* subsp. *laumondii* TT01.

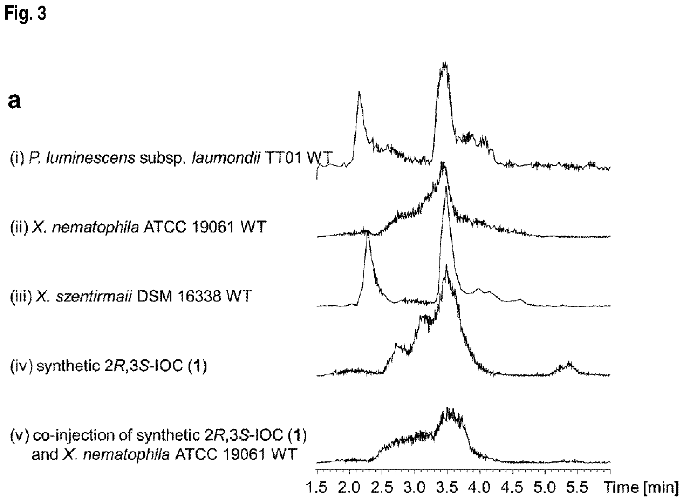

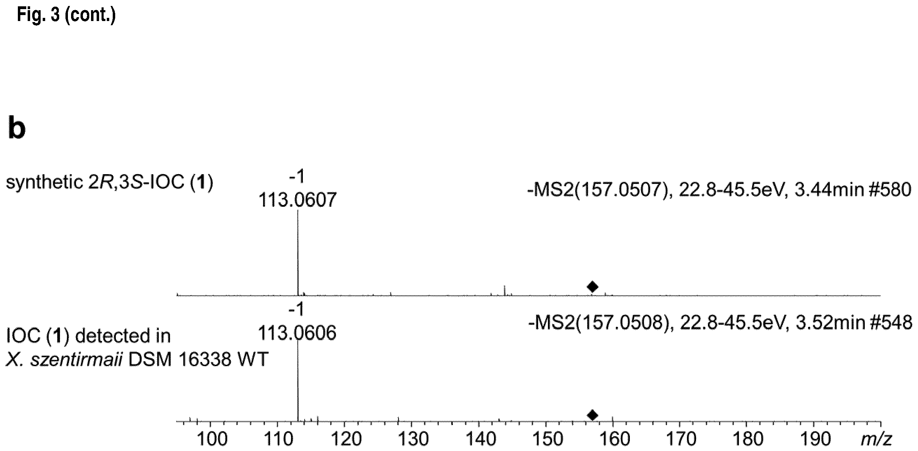

- **Fig. 3**: presents an HPLC-MS analysis of the compound **1** in different *Xenorhabdus* and *Photorhabdus* strains in Sf-900 medium. (**a**) 157.05075 [M - H]⁻ EICs of the culture supernatants of (i) *P. luminescens* subsp. *laumondii* TT01 WT, (ii) *Xenorhabdus nematophila* ATCC 19061 WT, and (iii) *Xenorhabdus szentirmaii* DSM 16338 WT, as well as (iv) synthetic 2*R*,3*S*-IOC (compound **1**) and (v) co-injection of synthetic 2*R*,3*S*-IOC (compound **1**) and the culture supernatant of *X. nematophila* ATCC 19061 WT. (**b**) Comparison of the MS/MS fragmentation patterns of the synthetic 2*R*,3*S*-IOC (compound **1**) and IOC (compound **1**) detected in the culture supernatant of *X. szentirmaii* DSM 16338 WT. The diamond indicates the parent ions. Representative data from three independent experiments are shown.

As explained above, the present invention relates to a compound of formula (I) 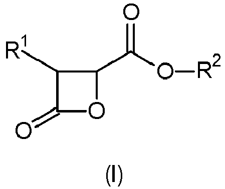 or a pharmaceutically acceptable salt thereof, for use in therapy.

The following detailed description of the compound of formula (I) relates to all embodiments of the present invention, and is also applicable to all salts (including pharmaceutically acceptable salts and agriculturally acceptable salts) of the compound of formula (I).

R¹ is selected from hydrogen, C₁₋₈ alkyl, C₂₋₈ alkenyl, C₂₋₈ alkynyl, -(C₁₋₆ alkylene)-O-R¹¹, -(C₁₋₆ alkylene)-S-R¹¹, -(C₁₋₆ alkylene)-N(R¹¹)-R¹¹, -(C₁₋₆ alkylene)-CO-R¹¹, -(C₁₋₆ alkylene)-COO-R¹¹, -(C₁₋₆ alkylene)-O-CO-(C₁₋₆ alkyl), -(C₁₋₆ alkylene)-CO-N(R¹¹)-R¹¹, -(C₁₋₆ alkylene )-N(R¹¹)-CO-(C₁₋₆ alkyl), -(C₁₋₆ alkylene)-CO-N(R¹¹)-O-R¹¹, -(C₁₋₆ alkylene)-O-CO-N(R¹¹)-R¹¹, -(C₁₋₆ alkylene)-N(R¹¹)-CO-N(R¹¹)-R¹¹, -(C₁₋₆ alkylene)-N(R¹¹)-C(=N-R¹¹)-N(R¹¹)-R¹¹, -(C₁₋₆ alkylene)-SO₃-R¹¹, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R³, wherein the said alkyl, said alkenyl, said alkynyl, and any alkylene group comprised in any of the aforementioned R¹ groups are each optionally substituted with one or more -OH, and further wherein, each R¹¹ is independently selected from hydrogen and C₁₋₆ alkyl.

R² is selected from hydrogen, C₁₋₆ alkyl, C₂₋₆ alkenyl, and C₂₋₆ alkynyl. Preferably, R² is hydrogen or C₁₋₄ alkyl. More preferably, R² is hydrogen, methyl or ethyl. Even more preferably, R² is hydrogen or methyl. Most preferably, R² is hydrogen.

Each R³ is independently selected from C₁₋₆ alkyl, C₂₋₆ alkenyl, C₂₋₆ alkynyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -S(C₁₋₆ alkylene)-SH, -S(C₁₋₆ alkylene)-S(C₁₋₆ alkyl), -(C₁₋₆ alkylene)-SH, -(C₁₋₆ alkylene)-S(C₁₋₆ alkyl), -NH₂, -NH(C₁₋₆ alkyl), -N(C₁₋₆ alkyl)(C₁₋₆ alkyl), -NH-OH, -N(C₁₋₆ alkyl)-OH, -NH-O(C₁₋₆ alkyl), -N(C₁₋₆ alkyl)-O(C₁₋₆ alkyl), =NH, =N(C₁₋₆ alkyl), halogen, C₁₋₆ haloalkyl, -O-(C₁₋₆ haloalkyl), -CN, -NO₂, -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), -SO-(C₁₋₆ alkyl), -(C₀₋₄ alkylene)-carbocyclyl, and -(C₀₋₄ alkylene)-heterocyclyl, wherein the carbocyclyl group in said -(C₀₋₄ alkylene)-carbocyclyl and the heterocyclyl group in said -(C₀₋₄ alkylene)-heterocyclyl are each optionally substituted with one or more groups independently selected from C₁₋₆ alkyl, C₂₋₆ alkenyl, C₂₋₆ alkynyl, halogen, C₁₋₆ haloalkyl, -O-(C₁₋₆ haloalkyl), -CN, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), and -SO-(C₁₋₆ alkyl).

Preferably, each R³ is independently selected from C₁₋₆ alkyl, C₂₋₆ alkenyl, C₂₋₆ alkynyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -S(C₁₋₆ alkylene)-SH, -S(C₁₋₆ alkylene)-S(C₁₋₆ alkyl), -(C₁₋₆ alkylene)-SH, -(C₁₋₆ alkylene)-S(C₁₋₆ alkyl), -NH₂, -NH(C₁₋₆ alkyl), -N(C₁₋₆ alkyl)(C₁₋₆ alkyl), -NH-OH, -N(C₁₋₆ alkyl)-OH, -NH-O(C₁₋₆ alkyl), -N(C₁₋₆ alkyl)-O(C₁₋₆ alkyl), =NH, =N(C₁₋₆ alkyl), halogen, C₁₋₆ haloalkyl, -O-(C₁₋₆ haloalkyl), -CN, -NO₂, -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), -SO-(C₁₋₆ alkyl), -(C₀₋₄ alkylene)-cycloalkyl, and -(C₀₋₄ alkylene)-heterocycloalkyl. More preferably, each R³ is independently selected from C₁₋₆ alkyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -NH₂, -NH(C₁₋₆ alkyl), -N(C₁₋₆ alkyl)(C₁₋₆ alkyl), halogen, C₁₋₆ haloalkyl, -O-(C₁₋₆ haloalkyl), and -CN.

Preferably, R¹ is selected from hydrogen, C₁₋₈ alkyl, C₂₋₈ alkenyl, C₂₋₈ alkynyl, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, -(C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, - (C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R³, and further wherein said alkyl, said alkenyl, said alkynyl, and any alkylene group in any of the aforementioned groups are each optionally substituted with one or more -OH.

More preferably, R¹ is selected from C₁₋₈ alkyl, C₂₋₈ alkenyl, C₂₋₈ alkynyl, phenyl, -(C₁₋₆ alkylene)-phenyl, cycloalkyl, - (C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R³, and further wherein said alkyl, said alkenyl, said alkynyl, and any alkylene group in any of the aforementioned groups are each optionally substituted with one or more -OH.

It is to be understood that the present invention specifically relates to each and every combination of features and embodiments described herein, including any combination of general and/or preferred features/embodiments.

In a first specific embodiment of the present invention, R¹ is C₁₋₆ alkyl. Particularly preferred C₁₋₆ alkyl groups are methyl, ethyl, n-propyl, isopropyl, n-butyl, isobutyl, sec-butyl, and tert-butyl. Thus, in this embodiment R¹ can be selected from methyl, ethyl, n-propyl, isopropyl, n-butyl, isobutyl, sec-butyl, and tert-butyl. Preferably, R¹ is selected from methyl, isopropyl, isobutyl, and sec-butyl. Even more preferably, R¹ is isopropyl.

In a second specific embodiment of the present invention, R¹ is selected from phenyl and -(C₁₋₆ alkylene)-phenyl, preferably R¹ is selected from phenyl, benzyl, and phenethyl. More preferably, R¹ is phenethyl.

In a third specific embodiment of the present invention, R¹ is a side chain of an amino acid, preferably of an α-amino acid (particularly a naturally occurring α-amino acid, including a proteinogenic α-amino acid or a non-proteinogenic α-amino acid). Examples of R¹ being a side chain of an amino acid are illustrated in Table 1 below.

**Table 1. Examples of R¹ being a side chain of an amino acid.**

| **R¹** | **Amino acid (side chain)** |
|---|---|
| hydrogen | Glycine |
| methyl | Alanine |
| ethyl | homoalanine |
| n-propyl | norvaline |
| isopropyl | valine |
| n-butyl | norleucine |
| isobutyl | leucine |
| sec-butyl | isoleucine |
| tert -butyl | terleucine |
| n-pentyl | homonorleucine |
| n-hexyl | 2-aminocaprylic acid |
| -C(-CH₃)(-OH)-CH₃ | β-hydroxyvaline |
| -CH(-OH)-CH(-CH₃)-CH₃ | β-hydroxyleucine |
| -CH₂-CH=CH-CH₃ | 2-amino-4-hexenoic acid |
| -CH₂-C=CH | propargylglycine |
| -CH(-OH)-C=CH | β-ethynylserine |
| phenyl | phenylglycine |
| 4-hydroxyphenyl | 4-hydroxyphenylglycine |
| 3,5-dihydroxyphenyl | 3,5-dihydroxyphenylglycine |
| -CH₂-phenyl | phenylalanine |
| -CH₂-(4-hydroxyphenyl) | tyrosine |
| -CH₂-(3,4-dihydroxyphenyl) | DOPA |
| -CH₂-(3-nitro-4-hydroxyphenyl) | 3-n i trotyrosi ne |
| -CH₂-(4-methoxyphenyl) | O-methyltyrosine |
| -CH₂-(3-hydroxy-4-methoxy-5-methylphenyl) | 3-OH-5-Me-OMe-tyrosine |
| -CH₂-(4-prenyloxyphenyl) | O-prenyltyrosine |
| -CH₂CH₂-phenyl | homophenylalanine |
| -CH₂CH₂-(4-hydroxyphenyl) | Homotyrosine |
| -CH(-OH)-CH₂-(4-nitrophenyl) | β-OH-p-NO₂-homophenylalanine |
| -CH₂-(1H-indol-3-yl) | tryptophan |
| -CH(-CH₃)-(1H-indol-3-yl) | β-methyl-tryptophan |
| -CH₂-(5-hydroxy-1H-indol-3-yl) | 5-hydroxytryptophan |
| -CH₂-(1-acetyl-6-hydroxy-1H-indol-3-yl) | 6-hydroxy-N-acetyltryptophan |
| -CH₂-(1-(1,1-dimethylallyl)-1H-indol-3-yl) | N-dimethylallyltryptophan |
| -CH(-CH₃)-(2-oxo-indolin-3-yl) | oxo-β-methyl-tryptophan |
| -CH₂-(4-nitro-1H-indol-3-yl) or -CH₂-(5-nitro-1H-indol-3-yl) | nitrated tryptophan |
| -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), or -CH₂-(7-bromo-1H-indol-3-yl) | halogenated tryptophan |
| -CH₂-(1H-imidazol-4-yl) | histidine |
| -CH₂-(5-mercapto-1-methyl-1H-imidazol-4-yl) | ovothiol |
| -CH₂-(3-furanyl) | 3-(3-furyl)-alanine |
| pyrrolidin-2-yl | tambroline |
| -CH₂-OH | serine |
| -CH₂CH₂-OH | homoserine |
| -CH₂CH₂-O-CH₃ | O-methyl-homoserine |
| -CH₂CH₂-O-CH₂CH₃ | O-ethyl-homoserine |
| -CH(-CH₃)-OH | threonine |
| -CH₂-SH | cysteine |
| -CH₂CH₂-SH | homocysteine |
| -CH₂CH₂-S-CH₃ | methionine |
| -CH₂CH₂-S-CH₂CH₃ | ethionine |
| -CH₂-SO₃H | cysteate |
| -CH₂-COOH | aspartate |
| -CH₂CH₂-COOH | glutamate |
| -CH₂-CO-NH₂ | asparagine |
| -CH₂CH₂-CO-NH₂ | glutamine |
| -(CH₂)₃-NH-CO-NH₂ | citrulline |
| -(CH₂)₂-CO-NH-OH | glutamate-γ-monohydroxamate |
| -CH₂-NH₂ | 2,3-diamino-propionic acid (Dap) |
| -(CH₂)₂-NH₂ | homo-Dap |
| -(CH₂)₃-NH₂ | ornithine |
| -(CH₂)₄-NH₂ | lysine |
| -CH(-CH₃)-(CH₂)₂-NH₂ | β-methylornithine |
| -C(-OH)-(CH₂)₃-NH₂ | 3-hydroxylysine |
| -CH₂-C(-OH)-(CH₂)₂-NH₂ | 4-hydroxylysine |
| -(CH₂)₂-C(-OH)-C(-CH₂-OH)-NH₂ | diamino-dihydroxy-heptanoic acid (DADH) |
| -(CH₂)₃-NH-C(=NH)-NH₂ | arginine |
| -CH(-CH₃)-(CH₂)₂-NH-C(=NH)-NH₂ | β-methylarginine |
| -C(-OH)-(CH₂)₂-NH-C(=NH)-NH₂ | 3-hydroxyargini ne |
| -CH₂-C(-OH)-CH₂-NH-C(=NH)-NH₂ | 4-hydroxyargini ne |
| -C(-OH)-C(-OH)-CH₂-NH-C(=NH)-NH₂ | 3,4-dihydroxyarginine |
| -(CH₂)₄-NH-C(=NH)-NH₂ | homoarginine |
| -C(-OH)-(CH₂)₃-NH-C(=NH)-NH₂ | 3-hydroxyhomoargi nine |
| -CH₂-C(-OH)-CH₂CH₂-NH-C(=NH)-NH₂ | 4-hydroxyhomoarginine |
| -CH(-OH)-CH(-OH)-CH₂-O-CO-NH₂ | carbamoylpolyoxamic acid |

In a fourth specific embodiment, R¹ is selected from hydrogen, methyl, ethyl, n-propyl, isopropyl, n-butyl, isobutyl, sec-butyl, tert-butyl, n-pentyl, n-hexyl, -CH₂-CH=CH-CH₃, -CH₂-C=CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, -CH₂-(2-nitrocycloprop-1-yl), -CH₂-OH, -CH₂CH₂-OH, -CH₂-O-CH₃, -CH₂CH₂-O-CH₃, -CH₂CH₂-O-CH₂CH₃, -CH(-CH₃)-OH, - C(-CH₃)(-OH)-CH₃, -CH(-OH)-CH(-CH₃)-CH₃, -CH(-OH)-CH(-CH₃)-CH₂-CH=CH-CH₃, -CH(-OH)-C=CH, -CH₂-SH, -CH₂CH₂-SH, -CH₂CH₂-S-CH₃, -CH₂CH₂-S-CH₂CH₃, -CH₂-SO₃H, -CH₂-COOH, -CH₂CH₂-COOH, -CH(-OH)-COOH, -CH(-OH)-CH₂-COOH, -CH(-CH₃)-COOH, -CH(-CH₃)-CH₂-COOH, -CH₂-CO-NH₂, -CH₂CH₂-CO-NH₂, -CH(-OH)-CO-NH₂, -CH₂-NH-CO-NH₂, -(CH₂)₂-NH-CO-NH₂, -(CH₂)₃-NH-CO-NH₂, -(CH₂)₂-CO-NH-OH, -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, -(CH₂)₂-C(-OH)-C(-CH₂-OH)-NH₂, -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, -CH₂-C(-OH)-CH₂CH₂-NH-C(=NH)-NH₂, and -CH(-OH)-CH(-OH)-CH₂-O-CO-NH₂.

In a fifth specific embodiment, R¹ is selected from hydrogen, methyl, ethyl, n-propyl, isopropyl, n-butyl, isobutyl, sec-butyl, tert-butyl, n-pentyl, n-hexyl, -CH₂-CH=CH-CH₃, -CH₂-C=CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, and -CH₂-(2-nitrocycloprop-1-yl).

In a sixth specific embodiment of the present invention, R¹ is hydrogen, which corresponds to a side chain of glycine.

In a seventh specific embodiment, R¹ is selected from C₁₋₈ alkyl. Preferably, R¹ is selected from methyl, ethyl, n-propyl, isopropyl, n-butyl, isobutyl, sec-butyl, tert-butyl, n-pentyl, and n-hexyl. In certain embodiments of the present invention, R¹ is methyl, which corresponds to a side chain of alanine. In a particular embodiment of the present invention, R¹ is ethyl, which corresponds to a side chain of homoalanine. In a further particular embodiment of the present invention, R¹ is n-propyl, which corresponds to a side chain of norvaline. In a further particular embodiment of the present invention, R¹ is isopropyl, which corresponds to a side chain of valine. In a further particular embodiment of the present invention, R¹ is n-butyl, which corresponds to a side chain of norleucine. In a further particular embodiment of the present invention, R¹ is isobutyl, which corresponds to a side chain of leucine. In a further particular embodiment of the present invention, R¹ is sec-butyl, which corresponds to a side chain of isoleucine. In a further particular embodiment of the present invention, R¹ is tert-butyl, which corresponds to a side chain of terleucine. In a further particular embodiment of the present invention, R¹ is n-pentyl, which corresponds to a side chain of homonorleucine. In a further particular embodiment of the present invention, R¹ is n-hexyl, which corresponds to a side chain of 2-aminocaprylic acid.

In an eighth specific embodiment of the present invention, R¹ is selected from C₂₋₈ alkenyl and C₂₋₈ alkynyl. Preferably, R¹ is selected from -CH₂-CH=CH-CH₃ and -CH₂-C=CH. In a particular embodiment of the present invention, R¹ is - CH₂-CH=CH-CH₃, which corresponds to a side chain of 2-amino-4-hexenoic acid. In a further particular embodiment of the present invention, R¹ is -CH₂-C=CH, which corresponds to a side chain of propargylglycine.

In a ninth specific embodiment of the present invention, R¹ is phenyl or -(C₁₋₆ alkylene)-phenyl, wherein said phenyl and the phenyl group in said -(C₁₋₆ alkylene)-phenyl are each optionally substituted with one or more groups R³. Preferably, R¹ is selected from phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), and -CH(-OH)-CH₂-(4-nitrophenyl).

In a particular embodiment of the present invention, R¹ is phenyl, which corresponds to a side chain of phenylglycine. In a further particular embodiment of the present invention, R¹ is 4-hydroxyphenyl, which corresponds to a side chain of 4-hydroxyphenylglycine. In a further particular embodiment of the present invention, R¹ is 3,5-dihydroxyphenyl, which corresponds to a side chain of 3,5-dihydroxyphenylglycine. In a further particular embodiment of the present invention, R¹ is -CH₂-phenyl, which corresponds to a side chain of phenylalanine. In a further particular embodiment of the present invention, R¹ is -CH₂-(4-hydroxyphenyl), which corresponds to a side chain of tyrosine. In a further particular embodiment of the present invention, R¹ is -CH₂-(3,4-dihydroxyphenyl), which corresponds to a side chain of DOPA. In a further particular embodiment of the present invention, R¹ is -CH₂-(3-nitro-4-hydroxyphenyl), which corresponds to a side chain of 3-nitrotyrosine. In a further particular embodiment of the present invention, R¹ is -CH₂-(4-methoxyphenyl) which corresponds to a side chain of O-methyltyrosine. In a further particular embodiment of the present invention, R¹ is -CH₂-(3-hydroxy-4-methoxy-5-methylphenyl) which corresponds to a side chain of 3-OH-5-Me-OMe-tyrosine. In a further particular embodiment of the present invention, R¹ is -CH₂-(4-prenyloxyphenyl) which corresponds to a side chain of O-prenyltyrosine. In a further particular embodiment of the present invention, R¹ is - CH₂CH₂-phenyl which corresponds to a side chain of homophenylalanine. In a further particular embodiment of the present invention, R¹ is -CH₂CH₂-(4-hydroxyphenyl) which corresponds to a side chain of homotyrosine. In a further particular embodiment of the present invention, R¹ is -CH(-OH)-CH₂-(4-nitrophenyl) which corresponds to a side chain of β-OH-p-NO₂-homophenylalanine.

In a tenth specific embodiment of the present invention, R¹ is -(C₀₋₆ alkylene)-heterocyclyl, wherein said heterocyclyl is optionally substituted with one or more groups R³. Preferably, the heterocyclyl in said -(C₀₋₆ alkylene)-heterocyclyl is 1H-indol-3-yl. Further preferably, R¹ is selected from -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), - CH₂-(7-chloro-1H-indol-3-yl), -CH₂-(7-bromo-1H-indol-3-yl), and -CH₂-(6,7-dichloro-1H-indol-3-yl). Thus, in a particular embodiment of the present invention, R¹ is -CH₂-(1H-indol-3-yl) which corresponds to a side chain of tryptophan. In a further particular embodiment of the present invention, R¹ is -CH(-CH₃)-(1H-indol-3-yl) which corresponds to a side chain of β-methyl-tryptophan. In a further particular embodiment of the present invention, R¹ is -CH₂-(5-hydroxy-1H-indol-3-yl) which corresponds to a side chain of 5-hydroxytryptophan. In a further particular embodiment of the present invention, R¹ is -CH₂-(1-acetyl-6-hydroxy-1H-indol-3-yl) which corresponds to a side chain of 6-hydroxy-N-acetyltryptophan. In a further particular embodiment of the present invention, R¹ is -CH₂-(1-(1,1-dimethylallyl)-1H-indol-3-yl) which corresponds to a side chain of N-dimethylallyltryptophan. In a further particular embodiment of the present invention, R¹ is -CH(-CH₃)-(2-oxo-indolin-3-yl) which corresponds to a side chain of oxo-β-methyl-tryptophan. In a further particular embodiment of the present invention, R¹ is -CH₂-(4-nitro-1H-indol-3-yl) or -CH₂-(5-nitro-1H-indol-3-yl) which corresponds to a side chain of nitrated tryptophan. In a further particular embodiment of the present invention, R¹ is -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), or -CH₂-(7-bromo-1H-indol-3-yl), which correspond to a side chain of halogenated tryptophan.

In an eleventh specific embodiment of the present invention, R¹ is -(C₀₋₆ alkylene)-heterocyclyl, wherein said heterocyclyl is optionally substituted with one or more groups R³, R¹ is preferably selected from -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), and 2-iminohexahydropyrimidin-4-yl. In a particular embodiment of the present invention, R¹ is -CH₂-(1H-imidazol-4-yl) which corresponds to a side chain of histidine. In a further particular embodiment of the present invention, R¹ is -CH₂-(5-mercapto-1-methyl-1H-imidazol-4-yl) which corresponds to a side chain of ovothiol. In a further particular embodiment of the present invention, R¹ is -CH₂-(3-furanyl) which corresponds to a side chain of 3-(3-furyl)-alanine. In a further particular embodiment of the present invention, R¹ is pyrrolidin-2-yl which corresponds to a side chain of tambroline.

In a twelfth specific embodiment of the present invention, R¹ is cycloalkyl or -(C₁₋₆ alkylene)-cycloalkyl, wherein said cycloalkyl and the cycloalkyl group in said -(C₁₋₆ alkylene)-cycloalkyl are each optionally substituted with one or more groups R³. Preferably, R¹ is cycloalkyl, preferably a cyclopropyl, which may be optionally substituted with one or more groups R³. Preferably, R¹ is selected from 1-methylcycloprop-1-yl, and -CH₂-(2-nitrocycloprop-1-yl).

In a thirteenth specific embodiment, R¹ is selected from C₁₋₈ alkyl, C₂₋₈ alkenyl, C₂₋₈ alkynyl, and -(C₁₋₆ alkylene)-OR¹¹, wherein said alkyl, said alkenyl, said alkynyl, and said alkylene group are each optionally substituted with one or more -OH, and further wherein each R¹¹ is independently selected from hydrogen and C₁₋₆ alkyl. Preferably, R¹ is selected from -CH₂-OH, -CH₂CH₂-OH, -CH₂-O-CH₃, -CH₂CH₂-O-CH₃, -CH₂CH₂-O-CH₂CH₃, -CH(-CH₃)-OH, -C(-CH₃)(-OH)-CH₃, -CH(-OH)-CH(-CH₃)-CH₃, -CH(-OH)-CH(-CH₃)-CH₂-CH=CH-CH₃, and -CH(-OH)-C=CH. In a particular embodiment of the present invention, R¹ is -C(-CH₃)(-OH)-CH₃, which corresponds to a side chain of β-hydroxyvaline. In a further particular embodiment of the present invention, R¹ is -CH(-OH)-CH(-CH₃)-CH₃, which corresponds to a side chain of β-hydroxyleucine. In a further particular embodiment of the present invention, R¹ is - CH(-OH)-C=CH, which corresponds to a side chain of β-ethynyiserine. In a further particular embodiment of the present invention, R¹ is -CH₂-OH which corresponds to a side chain of serine. In a further particular embodiment of the present invention, R¹ is -CH₂CH₂-OH which corresponds to a side chain of homoserine. In a further particular embodiment of the present invention, R¹ is -CH₂CH₂-O-CH₃ which corresponds to a side chain of O-methyl-homoserine. In a further particular embodiment of the present invention, R¹ is -CH₂CH₂-O-CH₂CH₃ which corresponds to a side chain of O-ethyl-homoserine. In a further particular embodiment of the present invention, R¹ is -CH(-CH₃)-OH which corresponds to a side chain of threonine.

In a fourteenth specific embodiment, R¹ is -(C₁₋₆ alkylene)-S-R¹¹, wherein R¹¹ is selected from hydrogen and C₁₋₆ alkyl. Preferably, R¹ is selected from -CH₂-SH, -CH₂CH₂-SH, -CH₂CH₂-S-CH₃, and -CH₂CH₂-S-CH₂CH₃. In a particular embodiment of the present invention, R¹ is -CH₂-SH which corresponds to a side chain of cysteine. In a further particular embodiment of the present invention, R¹ is -CH₂CH₂-SH which corresponds to a side chain of homocysteine. In a further particular embodiment of the present invention, R¹ is -CH₂CH₂-S-CH₃ which corresponds to a side chain of methionine. In a further particular embodiment of the present invention, R¹ is -CH₂CH₂-S-CH₂CH₃ which corresponds to a side chain of ethionine.

In a fifteenth specific embodiment of the present invention, R¹ is -(C₁₋₆ alkylene)-SO₃-R¹¹, wherein R¹¹ is selected from hydrogen and C₁₋₆ alkyl. Preferably, R¹ is -CH₂-SO₃H. In a particular embodiment of the present invention, R¹ is -CH₂-SO₃H which corresponds to a side chain of cysteate.

In a sixteenth specific embodiment of the present invention, R¹ is -(C₁₋₆ alkylene)-COO-R¹¹, wherein R¹¹ is selected from hydrogen and C₁₋₆ alkyl, wherein said alkyl, and said alkylene group are each optionally substituted with one or more -OH. Preferably, R¹¹ is hydrogen. Preferably, in certain embodiments R¹ is selected from -CH₂-COOH, -CH₂CH₂-COOH, -CH(-OH)-COOH, -CH(-OH)-CH₂-COOH, -CH(-CH₃)-COOH, -CH(-CH₃)-CH₂-COOH. In a particular embodiment of the present invention, R¹ is -CH₂-COOH, which corresponds to a side chain of aspartate.

In a seventeenth specific embodiment of the present invention, R¹ is -(C₁₋₆ alkylene)-CO-N(R¹¹)-R¹¹, wherein R¹¹ is selected from hydrogen and C₁₋₆ alkyl, and wherein said alkyl, and said alkylene group are each optionally substituted with one or more -OH. Preferably, R¹¹ is hydrogen. Preferably, in certain embodiments R¹ is selected from -CH₂-CO-NH₂, -CH₂CH₂-CO-NH₂, -CH(-OH)-CO-NH₂, and -CH₂-NH-CO-NH₂.

In an eighteenth specific embodiment of the present invention, R¹ is selected from -(C₁₋₆ alkylene)-CO-N(R¹¹)-O-R¹¹ and -(C₁₋₆ alkylene)-N(R¹¹)-CO-N(R¹¹)-R¹¹, wherein each R¹¹ is selected from hydrogen and C₁₋₆ alkyl. Preferably, R¹¹ is hydrogen. Furthermore, said alkylene group is optionally substituted with one or more -OH. Preferably, R¹ is selected from -(CH₂)₂-NH-CO-NH₂, -(CH₂)₃-NH-CO-NH₂, and -(CH₂)₂-CO-NH-OH.

In a nineteenth specific embodiment of the present invention, R¹ is -(C₁₋₆ alkylene)-N(R¹¹)-R¹¹, wherein each R¹¹ is selected from hydrogen and C₁₋₆ alkyl, and wherein said alkylene group is optionally substituted with one or more -OH. Preferably, R¹¹ is hydrogen. Preferably, R¹ is selected from -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, and -(CH₂)₂-C(-OH)-C(-CH₂-OH)-NH₂. In a particular embodiment of the present invention, R¹ is -(CH₂)₃-NH₂, which corresponds to the side chain of ornithine. In a further particular embodiment of the present invention, R¹ is -(CH₂)₄-NH₂, which corresponds to the side chain of lysine.

In a twentieth specific embodiment of the present invention, R¹ is -(C₁₋₆ alkylene)-N(R¹¹)-C(=N-R¹¹)-N(R¹¹)-R¹¹, wherein each R¹¹ is selected from hydrogen and C₁₋₆ alkyl, and wherein said alkylene group is optionally substituted with one or more -OH. Preferably, R¹¹ is hydrogen. Preferably, R¹ is selected from -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, and -CH₂-C(-OH)-CH₂CH₂-NH-C(=NH)-NH₂. In a particular embodiment R¹ is -(CH₂)₃-NH-C(=NH)-NH₂, which corresponds to the side chain of arginine.

In a twenty-first specific embodiment, R¹ is -(C₁₋₆ alkylene)-O-CO-N(R¹¹)-R¹¹, wherein each R¹¹ is selected from hydrogen and C₁₋₆ alkyl, and wherein said alkylene group is optionally substituted with one or more -OH. Preferably, R¹¹ is hydrogen. Preferably, R¹ is -CH(-OH)-CH(-OH)-CH₂-O-CO-NH₂.

Preferably, in the compound of formula (I), the groups -R¹ and -C(=O)-O-R² are present in trans configuration. More preferably, the compound of formula (I) has the following absolute configuration:

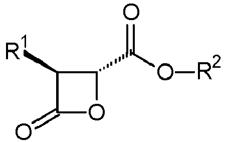

A particularly preferred compound of formula (I) for use in accordance with the present invention is any one of the following compounds or a salt (e.g., a pharmaceutically acceptable salt) thereof: 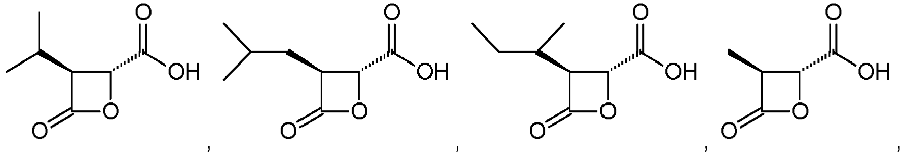 or

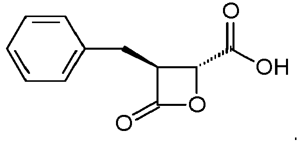

Even more preferably, the compound of formula (I) for use in accordance with the present invention compound is 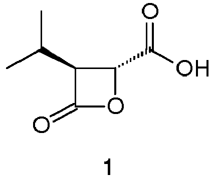 or a salt (e.g., a pharmaceutically acceptable salt) thereof.

The compounds of formula (I) can be prepared in accordance with, or in analogy to, the synthetic routes described in the examples section.

### Definitions

The following definitions apply throughout the present specification and the claims, unless specifically indicated otherwise.

The term "hydrocarbon group" refers to a group consisting of carbon atoms and hydrogen atoms.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₈ alkyl" denotes an alkyl group having 1 to 8 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₈ alkenyl" denotes an alkenyl group having 2 to 8 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₈ alkynyl" denotes an alkynyl group having 2 to 8 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₆ alkylene" denotes an alkylene group having 1 to 6 carbon atoms, and the term "C₀₋₆ alkylene" indicates that a covalent bond (corresponding to the option "C₀ alkylene") or a C₁₋₆ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, or cycloalkyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, or heterocycloalkyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members (e.g., cyclopropyl or cyclohexyl).

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "halogen" refers to fluoro (-F), chloro (-CI), bromo (-Br), or iodo (-I).

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

It is to be understood that wherever numerical ranges are provided/disclosed herein, all values and subranges encompassed by the respective numerical range are meant to be encompassed within the scope of the invention. Accordingly, the present invention specifically and individually relates to each value that falls within a numerical range disclosed herein, as well as each subrange encompassed by a numerical range disclosed herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

### Compounds of the invention

The scope of the invention embraces all pharmaceutically acceptable salt forms of the compounds of formula (I) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (I) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (I) is a hydrochloride salt. Accordingly, it is preferred that the compound of formula (I), including any one of the specific compounds of formula (I) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (I) is in the form of a hydrochloride salt.

The present invention also specifically relates to the compound of formula (I), including any one of the specific compounds of formula (I) described herein, in non-salt form.

Moreover, the scope of the invention embraces the compounds of formula (I) in any solvated form, including, e.g., solvates with water (i.e., as a hydrate) or solvates with organic solvents such as, e.g., methanol, ethanol, isopropanol, acetic acid, ethyl acetate, ethanolamine, DMSO, or acetonitrile. All physical forms, including any amorphous or crystalline forms (i.e., polymorphs), of the compounds of formula (I) are also encompassed within the scope of the invention. It is to be understood that such solvates and physical forms of pharmaceutically acceptable salts of the compounds of the formula (I) are likewise embraced by the invention.

Furthermore, the compounds of formula (I) may exist in the form of different isomers, in particular stereoisomers (including, e.g., geometric isomers (or cis/trans isomers), enantiomers and diastereomers) or tautomers (including, in particular, prototropic tautomers, such as keto/enol tautomers or thione/thiol tautomers). All such isomers of the compounds of formula (I) are contemplated as being part of the present invention, either in admixture or in pure or substantially pure form. As for stereoisomers, the invention embraces the isolated optical isomers of the compounds according to the invention as well as any mixtures thereof (including, in particular, racemic mixtures/racemates and non-racemic mixtures). The racemates can be resolved by physical methods, such as, e.g., fractional crystallization, separation or crystallization of diastereomeric derivatives, or separation by chiral column chromatography. The individual optical isomers can also be obtained from the racemates via salt formation with an optically active acid followed by crystallization. The present invention further encompasses any tautomers of the compounds of formula (I). It will be understood that some compounds may exhibit tautomerism. In such cases, the formulae provided herein expressly depict only one of the possible tautomeric forms. The formulae and chemical names as provided herein are intended to encompass any tautomeric form of the corresponding compound and not to be limited merely to the specific tautomeric form depicted by the drawing or identified by the name of the compound.

The scope of the invention also embraces compounds of formula (I), in which one or more atoms are replaced by a specific isotope of the corresponding atom. For example, the invention encompasses compounds of formula (I), in which one or more hydrogen atoms (or, e.g., all hydrogen atoms) are replaced by deuterium atoms (i.e., ²H; also referred to as "D"). Accordingly, the invention also embraces compounds of formula (I) which are enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in the compounds of formula (I) can be increased using deuteration techniques known in the art. For example, a compound of formula (I) or a reactant or precursor to be used in the synthesis of the compound of formula (I) can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that the compound of formula (I) is not enriched in deuterium. Accordingly, the presence of naturally occurring hydrogen atoms or ¹H hydrogen atoms in the compounds of formula (I) is preferred.

The present invention also embraces compounds of formula (I), in which one or more atoms are replaced by a positron-emitting isotope of the corresponding atom, such as, e.g., ¹⁸F, ¹¹C, ¹³N, ¹⁵O, ⁷⁶Br, ⁷⁷Br, ¹²⁰I and/or ¹²⁴I. Such compounds can be used as tracers, trackers or imaging probes in positron emission tomography (PET). The invention thus includes (i) compounds of formula (I), in which one or more fluorine atoms (or, e.g., all fluorine atoms) are replaced by ¹⁸F atoms, (ii) compounds of formula (I), in which one or more carbon atoms (or, e.g., all carbon atoms) are replaced by ¹¹C atoms, (iii) compounds of formula (I), in which one or more nitrogen atoms (or, e.g., all nitrogen atoms) are replaced by ¹³N atoms, (iv) compounds of formula (I), in which one or more oxygen atoms (or, e.g., all oxygen atoms) are replaced by ¹⁵O atoms, (v) compounds of formula (I), in which one or more bromine atoms (or, e.g., all bromine atoms) are replaced by ⁷⁶Br atoms, (vi) compounds of formula (I), in which one or more bromine atoms (or, e.g., all bromine atoms) are replaced by ⁷⁷Br atoms, (vii) compounds of formula (I), in which one or more iodine atoms (or, e.g., all iodine atoms) are replaced by ¹²⁰I atoms, and (viii) compounds of formula (I), in which one or more iodine atoms (or, e.g., all iodine atoms) are replaced by ¹²⁴I atoms. In general, it is preferred that none of the atoms in the compounds of formula (I) are replaced by specific isotopes.

### Pharmaceutical compositions

The present invention relates to a pharmaceutical composition comprising a compound of formula (I) or a pharmaceutically acceptable salt thereof of the present invention as defined herein, and a pharmaceutically acceptable excipient. The present invention further relates to the said pharmaceutical composition comprising a compound of formula (I) or a pharmaceutically acceptable salt thereof of the present invention, and a pharmaceutically acceptable excipient for use in therapy.

The compounds and/or chemical entities provided herein may be administered as compounds *per se* or may be formulated as medicaments. The medicaments/pharmaceutical compositions may optionally comprise one or more pharmaceutically acceptable excipients, such as carriers, diluents, fillers, disintegrants, lubricating agents, binders, colorants, pigments, stabilizers, preservatives, antioxidants, and/or solubility enhancers.

The pharmaceutical compositions may comprise one or more solubility enhancers, such as, e.g., poly(ethylene glycol), including poly(ethylene glycol) having a molecular weight in the range of about 200 to about 5,000 Da (e.g., PEG 200, PEG 300, PEG 400, or PEG 600), ethylene glycol, propylene glycol, glycerol, a non-ionic surfactant, tyloxapol, polysorbate 80, macrogol-15-hydroxystearate (e.g., Kolliphor^{®} HS 15, CAS 70142-34-6), a phospholipid, lecithin, dimyristoyl phosphatidylcholine, dipalmitoyl phosphatidylcholine, distearoyl phosphatidylcholine, a cyclodextrin, α-cyclodextrin, β-cyclodextrin, y-cyclodextrin, hydroxyethyl-β-cyclodextrin, hydroxypropyl-β-cyclodextrin, hydroxyethyl-γ-cyclodextrin, hydroxypropyl-γ-cyclodextrin, dihydroxypropyl-β-cyclodextrin, sulfobutylether-β-cyclodextrin, sulfobutylether-γ-cyclodextrin, glucosyl-α-cyclodextrin, glucosyl-β-cyclodextrin, diglucosyl-β-cyclodextrin, maltosyl-α-cyclodextrin, maltosyl-β-cyclodextrin, maltosyl-γ-cyclodextrin, maltotriosyl-β-cyclodextrin, maltotriosyl-γ-cyclodextrin, dimaltosyl-β-cyclodextrin, methyl-β-cyclodextrin, a carboxyalkyl thioether, hydroxypropyl methylcellulose, hydroxypropylcellulose, polyvinylpyrrolidone, a vinyl acetate copolymer, vinyl pyrrolidone, sodium lauryl sulfate, dioctyl sodium sulfosuccinate, or any combination thereof.

The pharmaceutical compositions may also comprise one or more preservatives, particularly one or more antimicrobial preservatives, such as, e.g., benzyl alcohol, chlorobutanol, 2-ethoxyethanol, m-cresol, chlorocresol (e.g., 2-chloro-3-methyl-phenol or 4-chloro-3-methyl-phenol), benzalkonium chloride, benzethonium chloride, benzoic acid (or a pharmaceutically acceptable salt thereof), sorbic acid (or a pharmaceutically acceptable salt thereof), chlorhexidine, thimerosal, or any combination thereof.

The pharmaceutical compositions can be formulated by techniques known to the person skilled in the art, such as the techniques published in "Remington: The Science and Practice of Pharmacy", Pharmaceutical Press, 22nd edition. The pharmaceutical compositions can be formulated as dosage forms for oral, parenteral, such as intramuscular, intravenous, subcutaneous, intradermal, intraarterial, intracardial, rectal, nasal, topical, aerosol or vaginal administration. Dosage forms for oral administration include coated and uncoated tablets, soft gelatin capsules, hard gelatin capsules, lozenges, troches, solutions, emulsions, suspensions, syrups, elixirs, powders and granules for reconstitution, dispersible powders and granules, medicated gums, chewing tablets and effervescent tablets. Dosage forms for parenteral administration include solutions, emulsions, suspensions, dispersions and powders and granules for reconstitution. Emulsions are a preferred dosage form for parenteral administration. Dosage forms for rectal and vaginal administration include suppositories and ovula. Dosage forms for nasal administration can be administered via inhalation and insufflation, for example by a metered inhaler. Dosage forms for topical administration include creams, gels, ointments, salves, patches and transdermal delivery systems.

The compounds of formula (I) or the pharmaceutically acceptable salts thereof or the above described pharmaceutical compositions comprising the compound of formula (I) or the pharmaceutically acceptable salt thereof may be administered to a subject by any convenient route of administration, whether systemically/peripherally or at the site of desired action, including but not limited to one or more of: oral (e.g., as a tablet, capsule, or as an ingestible solution), topical (e.g., transdermal, intranasal, ocular, buccal, and sublingual), parenteral (e.g., using injection techniques or infusion techniques, and including, for example, by injection, e.g., subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, or intrasternal by, e.g., implant of a depot, for example, subcutaneously or intramuscularly), pulmonary (e.g., by inhalation or insufflation therapy using, e.g., an aerosol, e.g., through mouth or nose), gastrointestinal, intrauterine, intraocular, subcutaneous, ophthalmic (including intravitreal or intracameral), rectal, or vaginal administration.

If said compounds or pharmaceutically acceptable salts or pharmaceutical compositions are administered parenterally, then examples of such administration include one or more of: intravenously, intraarterially, intraperitoneally, intrathecally, intraventricularly, intraurethrally, intrasternally, intracardially, intracranially, intramuscularly or subcutaneously administering the compounds or pharmaceutical compositions, and/or by using infusion techniques. For parenteral administration, the compounds are best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well known to those skilled in the art.

Said compounds or pharmaceutically acceptable salts or pharmaceutical compositions can also be administered orally in the form of tablets, capsules, ovules, elixirs, solutions or suspensions, which may contain flavoring or coloring agents, for immediate-, delayed-, modified-, sustained-, pulsed- or controlled-release applications.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia. Additionally, lubricating agents such as magnesium stearate, stearic acid, glyceryl behenate and talc may be included. Solid compositions of a similar type may also be employed as fillers in gelatin capsules. Preferred excipients in this regard include lactose, starch, a cellulose, or high molecular weight polyethylene glycols. For aqueous suspensions and/or elixirs, the agent may be combined with various sweetening or flavoring agents, coloring matter or dyes, with emulsifying and/or suspending agents and with diluents such as water, ethanol, propylene glycol and glycerin, and combinations thereof.

For oral administration, the compounds, the pharmaceutically acceptable salts or the pharmaceutical compositions are preferably administered by oral ingestion, particularly by swallowing. The compounds or pharmaceutical compositions can thus be administered to pass through the mouth into the gastrointestinal tract, which can also be referred to as "oral-gastrointestinal" administration.

Alternatively, said compounds, pharmaceutically acceptable salts or pharmaceutical compositions can be administered in the form of a suppository or pessary, or may be applied topically in the form of a gel, hydrogel, lotion, solution, cream, ointment or dusting powder. The compounds of the present invention may also be dermally or transdermally administered, for example, by the use of a skin patch.

Said compounds, pharmaceutically acceptable salts or pharmaceutical compositions may also be administered by sustained release systems. Suitable examples of sustained-release compositions include semi-permeable polymer matrices in the form of shaped articles, e.g., films, or microcapsules. Sustained-release matrices include, e.g., polylactides, copolymers of L-glutamic acid and gamma-ethyl-L-glutamate, poly(2-hydroxyethyl methacrylate), ethylene vinyl acetate, or poly-D-(-)-3-hydroxybutyric acid. Sustained-release pharmaceutical compositions also include liposomally entrapped compounds. The present invention thus also relates to liposomes containing a compound of the invention or a pharmaceutically acceptable salt thereof.

Said compounds, pharmaceutically acceptable salts or pharmaceutical compositions may also be administered by the pulmonary route, rectal routes, or the ocular route. For ophthalmic use, they can be formulated as micronized suspensions in isotonic, pH adjusted, sterile saline, or, preferably, as solutions in isotonic, pH adjusted, sterile saline, optionally in combination with a preservative such as a benzalkonium chloride. Alternatively, they may be formulated in an ointment such as petrolatum.

It is also envisaged to prepare dry powder formulations of the compounds of formula (I) or the pharmaceutically acceptable salt thereof for pulmonary administration, particularly inhalation. Such dry powders may be prepared by spray drying under conditions which result in a substantially amorphous glassy or a substantially crystalline bioactive powder. Accordingly, dry powders of the compounds of the present invention can be made according to an emulsification/spray drying process.

For topical application to the skin, said compounds, pharmaceutically acceptable salts or pharmaceutical compositions can be formulated as a suitable ointment containing the active compound suspended or dissolved in, for example, a mixture with one or more of the following: mineral oil, liquid petrolatum, white petrolatum, propylene glycol, emulsifying wax and water. Alternatively, they can be formulated as a suitable lotion or cream, suspended or dissolved in, for example, a mixture of one or more of the following: mineral oil, sorbitan monostearate, a polyethylene glycol, liquid paraffin, polysorbate 60, cetyl esters wax, 2-octyldodecanol, benzyl alcohol and water.

The present invention thus relates to the compounds, pharmaceutically acceptable salts or the pharmaceutical compositions provided herein, wherein the corresponding compound or pharmaceutical composition is to be administered by any one of: an oral route; topical route, including by transdermal, intranasal, ocular, buccal, or sublingual route; parenteral route using injection techniques or infusion techniques, including by subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, intrasternal, intraventricular, intraurethral, or intracranial route; pulmonary route, including by inhalation or insufflation therapy; gastrointestinal route; intrauterine route; intraocular route; subcutaneous route; ophthalmic route, including by intravitreal, or intracameral route; rectal route; or vaginal route. Preferred routes of administration are oral administration or parenteral administration. For each of the compounds or pharmaceutical compositions provided herein, it is particularly preferred that the respective compound or pharmaceutical composition is to be administered orally (particularly by oral ingestion).

Typically, a physician will determine the actual dosage which will be most suitable for an individual subject. The specific dose level and frequency of dosage for any particular individual subject may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the individual subject undergoing therapy.

A proposed, yet non-limiting dose of the compounds according to the invention for oral administration to a human (of approximately 70 kg body weight) may be 0.05 to 2000 mg, preferably 0.1 mg to 1000 mg, of the active ingredient per unit dose. The unit dose may be administered, e.g., 1 to 3 times per day. The unit dose may also be administered 1 to 7 times per week, e.g., with not more than one administration per day. It will be appreciated that it may be necessary to make routine variations to the dosage depending on the age and weight of the patient/subject as well as the severity of the condition to be treated. The precise dose and also the route of administration will ultimately be at the discretion of the attendant physician or veterinarian.

The compound of formula (I) or the pharmaceutically acceptable salt thereof is useful in the treatment or prevention of cancer or an infectious disease. Thus, the present invention relates to the compound of formula (I) or the pharmaceutically acceptable salt thereof for use in the treatment or prevention of cancer or an infectious disease.

The compound of formula (I) or the pharmaceutically acceptable salt thereof or a pharmaceutical composition comprising the compound of formula (I) or the pharmaceutically acceptable salt thereof can be administered in monotherapy (e.g., without concomitantly administering any further therapeutic agents, or without concomitantly administering any further therapeutic agents against the same disease that is to be treated or prevented with the compound of formula (I)).

However, the compound of formula (I), the pharmaceutically acceptable salt thereof or a pharmaceutical composition comprising the compound of formula (I) or the pharmaceutically acceptable salt thereof can also be administered in combination with one or more further therapeutic agents. If the compound of formula (I) is used in combination with a second therapeutic agent active against the same disease or condition, the dose of each compound may differ from that when the corresponding compound is used alone, in particular, a lower dose of each compound may be used. The combination of the compound of formula (I) with one or more further therapeutic agents may comprise the simultaneous/concomitant administration of the compound of formula (I) and the further therapeutic agent(s) (either in a single pharmaceutical formulation or in separate pharmaceutical formulations), or the sequential/separate administration of the compound of formula (I) and the further therapeutic agent(s). If administration is sequential, either the compound of formula (I) according to the invention or the one or more further therapeutic agents may be administered first. If administration is simultaneous, the one or more further therapeutic agents may be included in the same pharmaceutical formulation as the compound of formula (I), or they may be administered in two or more different (separate) pharmaceutical formulations.

In certain embodiments, the present invention relates to the compound of formula (I) or the pharmaceutically acceptable salt thereof for use in the treatment or prevention of cancer. Preferably, the cancer is selected from lung cancer, renal cancer, gastro-intestinal cancer, stomach cancer, colorectal cancer, colon cancer, malignant familial adenomatous polyposis, anal cancer, genitourinary cancer, bladder cancer, liver cancer, pancreatic cancer, ovarian cancer, cervical cancer, endometrial cancer, vaginal cancer, vulvar cancer, prostate cancer, testicular cancer, biliary tract cancer, hepatobiliary cancer, neuroblastoma, brain cancer, breast cancer, head and/or neck cancer, skin cancer, melanoma, Merkel-cell carcinoma, epidermoid cancer, squamous cell carcinoma, bone cancer, fibrosarcoma, Ewing's sarcoma, malignant mesothelioma, esophageal cancer, laryngeal cancer, mouth cancer, thymoma, neuroendocrine cancer, hematological cancer, leukemia, lymphoma, and multiple myeloma. Even more preferably, the cancer is multiple myeloma.

Preferably, in the context of the treatment or prevention of cancer, the one or more further therapeutic agents to be administered in combination with a compound of the present invention are anticancer drugs. The anticancer drug(s) to be administered in combination with a compound of formula (I) or the pharmaceutically acceptable salt thereof according to the invention may, e.g., be selected from: a tumor angiogenesis inhibitor (e.g., a protease inhibitor, an epidermal growth factor receptor kinase inhibitor, or a vascular endothelial growth factor receptor kinase inhibitor); a cytotoxic drug (e.g., an antimetabolite, such as purine and pyrimidine analog antimetabolites); an antimitotic agent (e.g., a microtubule stabilizing drug or an antimitotic alkaloid); a platinum coordination complex; an anti-tumor antibiotic; an alkylating agent (e.g., a nitrogen mustard or a nitrosourea); an endocrine agent (e.g., an adrenocorticosteroid, an androgen, an anti-androgen, an estrogen, an anti-estrogen, an aromatase inhibitor, a gonadotropin-releasing hormone agonist, or a somatostatin analog); or a compound that targets an enzyme or receptor that is overexpressed and/or otherwise involved in a specific metabolic pathway that is deregulated (or misregulated) in the tumor cell (e.g., ATP and GTP phosphodiesterase inhibitors, histone deacetylase inhibitors, protein kinase inhibitors (such as serine, threonine and tyrosine kinase inhibitors, e.g., Abelson protein tyrosine kinase inhibitors) and the various growth factors, their receptors and corresponding kinase inhibitors (such as epidermal growth factor receptor kinase inhibitors, vascular endothelial growth factor receptor kinase inhibitors, fibroblast growth factor inhibitors, insulin-like growth factor receptor inhibitors and platelet-derived growth factor receptor kinase inhibitors)); methionine, aminopeptidase inhibitors, proteasome inhibitors, cyclooxygenase inhibitors (e.g., cyclooxygenase-1 or cyclooxygenase-2 inhibitors), topoisomerase inhibitors (e.g., topoisomerase I inhibitors or topoisomerase II inhibitors), poly ADP ribose polymerase inhibitors (PARP inhibitors), and epidermal growth factor receptor (EGFR) inhibitors/antagonists.

An alkylating agent which can be used as an anticancer drug in combination with the compound of formula (I) or the pharmaceutically acceptable salt thereof of the present invention may be, for example, a nitrogen mustard (such as cyclophosphamide, mechlorethamine (chlormethine), uramustine, melphalan, chlorambucil, ifosfamide, bendamustine, or trofosfamide), a nitrosourea (such as carmustine, streptozocin, fotemustine, lomustine, nimustine, prednimustine, ranimustine, or semustine), an alkyl sulfonate (such as busulfan, mannosulfan, or treosulfan), an aziridine (such as hexamethylmelamine (altretamine), triethylenemelamine, ThioTEPA (N,N'N'-triethylenethiophosphoramide), carboquone, or triaziquone), a hydrazine (such as procarbazine), a triazene (such as dacarbazine), or an imidazotetrazine (such as temozolomide).

A platinum coordination complex which can be used as an anticancer drug in combination with the compound of formula (I) or the pharmaceutically acceptable salt thereof of the present invention may be, for example, cisplatin, carboplatin, nedaplatin, oxaliplatin, satraplatin, or triplatin tetranitrate.

A cytotoxic drug which can be used as an anticancer drug in combination with the compound of formula (I) or the pharmaceutically acceptable salt thereof of the present invention may be, for example, an antimetabolite, including folic acid analogue antimetabolites (such as aminopterin, methotrexate, pemetrexed, or raltitrexed), purine analogue antimetabolites (such as cladribine, clofarabine, fludarabine, 6-mercaptopurine (including its prodrug form azathioprine), pentostatin, or 6-thioguanine), and pyrimidine analogue antimetabolites (such as cytarabine, decitabine, 5-fluorouracil (including its prodrug forms capecitabine and tegafur), floxuridine, gemcitabine, enocitabine, or sapacitabine).

An antimitotic agent which can be used as an anticancer drug in combination with the compound of formula (I) or the pharmaceutically acceptable salt thereof of the present invention may be, for example, a taxane (such as docetaxel, larotaxel, ortataxel, paclitaxel/taxol, tesetaxel, or nab-paclitaxel (e.g., Abraxane^{®})), a Vinca alkaloid (such as vinblastine, vincristine, vinflunine, vindesine, or vinorelbine), an epothilone (such as epothilone A, epothilone B, epothilone C, epothilone D, epothilone E, or epothilone F) or an epothilone B analogue (such as ixabepilone/azaepothilone B).

An anti-tumor antibiotic which can be used as an anticancer drug in combination with the compound of formula (I) or the pharmaceutically acceptable salt thereof of the present invention may be, for example, an anthracycline (such as aclarubicin, daunorubicin, doxorubicin, epirubicin, idarubicin, amrubicin, pirarubicin, valrubicin, or zorubicin), an anthracenedione (such as mitoxantrone, or pixantrone) or an anti-tumor antibiotic isolated from Streptomyces (such as actinomycin (including actinomycin D), bleomycin, mitomycin (including mitomycin C), or plicamycin).

A tyrosine kinase inhibitor which can be used as an anticancer drug in combination with the compound of formula (I) or the pharmaceutically acceptable salt thereof of the present invention may be, for example, axitinib, bosutinib, cediranib, dasatinib, erlotinib, gefitinib, imatinib, lapatinib, lestaurtinib, nilotinib, semaxanib, sorafenib, sunitinib, axitinib, nintedanib, ponatinib, vandetanib, or vemurafenib.

A topoisomerase inhibitor which can be used as an anticancer drug in combination with the compound of formula (I) or the pharmaceutically acceptable salt thereof of the present invention may be, for example, a topoisomerase I inhibitor (such as irinotecan, topotecan, camptothecin, belotecan, rubitecan, or lamellarin D) or a topoisomerase II inhibitor (such as amsacrine, etoposide, etoposide phosphate, teniposide, or doxorubicin).

A PARP inhibitor which can be used as an anticancer drug in combination with the compound of formula (I) or the pharmaceutically acceptable salt thereof of the present invention may be, for example, niraparib, olaparib, rucaparib, talazoparib, veliparib, pamiparib (BGB-290), BMN-673, CEP 9722, MK 4827, E7016, or 3-aminobenzamide.

An EGFR inhibitor/antagonist which can be used as an anticancer drug in combination with the compound of formula (I) or the pharmaceutically acceptable salt thereof of the present invention may be, for example, gefitinib, erlotinib, lapatinib, afatinib, neratinib, osimertinib, brigatinib, dacomitinib, vandetanib, pelitinib, canertinib, icotinib, poziotinib, ABT-414, AV-412, PD 153035, PKI-166, BMS-690514, CUDC-101, AP26113, XL647, cetuximab, panitumumab, zalutumumab, nimotuzumab, or matuzumab.

Further anticancer drugs may also be used in combination with the compound of formula (I) or the pharmaceutically acceptable salt thereof of the present invention. The anticancer drugs may comprise biological or chemical molecules, like TNF-related apoptosis-inducing ligand (TRAIL), tamoxifen, amsacrine, bexarotene, estramustine, irofulven, trabectedin, cetuximab, panitumumab, tositumomab, alemtuzumab, bevacizumab, edrecolomab, gemtuzumab, alvocidib, seliciclib, aminolevulinic acid, methyl aminolevulinate, efaproxiral, porfimer sodium, talaporfin, temoporfin, verteporfin, alitretinoin, tretinoin, anagrelide, arsenic trioxide, atrasentan, bortezomib, carmofur, celecoxib, demecolcine, elesclomol, elsamitrucin, etoglucid, lonidamine, lucanthone, masoprocol, mitobronitol, mitoguazone, mitotane, oblimersen, omacetaxine, sitimagene, ceradenovec, tegafur, testolactone, tiazofurine, tipifarnib, vorinostat, iniparib, or copanlisib.

Also biological drugs, like antibodies, antibody fragments, antibody constructs (for example, single-chain constructs), and/or modified antibodies (like CDR-grafted antibodies, humanized antibodies, "fully human" antibodies, etc.) directed against cancer or tumor markers/factors/cytokines involved in proliferative diseases can be employed in cotherapy approaches with the compound of formula (I) or the pharmaceutically acceptable salt thereof of the present invention. Examples of such biological molecules are anti-HER2 antibodies (e.g. trastuzumab, Herceptin^{®}), anti-CD20 antibodies (e.g. Rituximab, Rituxan^{®}, MabThera^{®}, Reditux^{®}), anti-CD19/CD3 constructs (see, e.g., EP1071752) and anti-TNF antibodies (see, e.g., Taylor PC, Curr Opin Pharmacol, 2003, 3(3):323-328). Further antibodies, antibody fragments, antibody constructs and/or modified antibodies to be used in cotherapy approaches with the compounds of the invention can be found, e.g., in: Taylor PC, Curr Opin Pharmacol, 2003, 3(3):323-328; or Roxana A, Maedica, 2006, 1(1):63-65.

An anticancer drug which can be used in combination with the compound of formula (I) or the pharmaceutically acceptable salt thereof of the present invention may, for example, be an immunooncology therapeutic (such as an antibody (e.g., a monoclonal antibody or a polyclonal antibody), an antibody fragment, an antibody construct (e.g., a single-chain construct), or a modified antibody (e.g., a CDR-grafted antibody, a humanized antibody, or a "fully human" antibody) targeting any one of CTLA-4, PD-1, PD-L1, TIM3, LAG3, OX40, CSF1R, IDO, or CD40. Such immunooncology therapeutics include, e.g., an anti-CTLA-4 antibody (particularly an antagonistic or pathway-blocking anti-CTLA-4 antibody; e.g., ipilimumab or tremelimumab), an anti-PD-1 antibody (particularly an antagonistic or pathway-blocking anti-PD-1 antibody; e.g., nivolumab (BMS-936558), pembrolizumab (MK-3475), pidilizumab (CT-011), AMP-224, or APE02058), an anti-PD-L1 antibody (particularly a pathway-blocking anti-PD-L1 antibody; e.g., BMS-936559, MEDI4736, MPDL3280A (RG7446), MDX-1105, or MEDI6469), an anti-TIM3 antibody (particularly a pathway-blocking anti-TIM3 antibody), an anti-LAG3 antibody (particularly an antagonistic or pathway-blocking anti-LAG3 antibody; e.g., BMS-986016, IMP701, or IMP731), an anti-OX40 antibody (particularly an agonistic anti-OX40 antibody; e.g., MEDI0562), an anti-CSF1R antibody (particularly a pathway-blocking anti-CSF1R antibody; e.g., IMC-CS4 or RG7155), an anti-IDO antibody (particularly a pathway-blocking anti-IDO antibody), or an anti-CD40 antibody (particularly an agonistic anti-CD40 antibody; e.g., CP-870,893 or Chi Lob 7/4). Further immunooncology therapeutics are known in the art and are described, e.g., in: Kyi C et al., FEBS Lett, 2014, 588(2):368-76; Intlekofer AM et al., J Leukoc Biol, 2013, 94(1):25-39; Callahan MK et al., J Leukoc Biol, 2013, 94(1):41-53; Ngiow SF et al., Cancer Res, 2011, 71(21):6567-71; and Blattman JN et al., Science, 2004, 305(5681):200-5.

The combinations referred to above may conveniently be presented for use in the form of a pharmaceutical formulation. The individual components of such combinations may be administered either sequentially or simultaneously/concomitantly in separate or combined pharmaceutical formulations by any convenient route. When administration is sequential, either the compound of the present invention (i.e., the compound of formula (I) or a pharmaceutically acceptable salt thereof) or the further therapeutic agent(s) may be administered first. When administration is simultaneous, the combination may be administered either in the same pharmaceutical composition or in different pharmaceutical compositions. When combined in the same formulation, it will be appreciated that the two or more compounds must be stable and compatible with each other and the other components of the formulation. When formulated separately, they may be provided in any convenient formulation.

The compounds of formula (I) or the pharmaceutically acceptable salts thereof as described herein can also be administered in combination with physical therapy, such as radiotherapy. Radiotherapy may commence before, after, or simultaneously with administration of the compounds of the invention. For example, radiotherapy may commence about 1 to 10 minutes, about 1 to 10 hours, or about 24 to 72 hours after administration of the compound of formula (I). The subject/patient is exposed to radiation, preferably gamma radiation, whereby the radiation may be provided in a single dose or in multiple doses that are administered over several hours, days and/or weeks. Gamma radiation may be delivered according to standard radiotherapeutic protocols using standard dosages and regimens.

Moreover, the compounds of formula (I) or the pharmaceutically acceptable salt thereof - either in combination with one or more further anticancer agents (including any of the exemplary anticancer agents described above) or without any further anticancer agents - can also be administered in combination with an antiemetic agent. The antiemetic agent may, for example, be selected from alosetron, azasetron, bemesetron, cilansetron, clozapine, dazopride, dolasetron, granisetron, lerisetron, metoclopramide, mianserin, mirtazapine, olanzapine, ondansetron, palonosetron (e.g., palonosetron alone, or palonosetron in combination with netupitant), quetiapine, ramosetron, ricasetron, tropisetron, zatosetron, clozapine, cyproheptadine, hydroxyzine, olanzapine, risperidone, ziprasidone, dronabinol, nabilone, tetrahydrocannabinol, alizapride, bromopride, chlorpromazine, clebopride, domperidone, haloperidol, hydroxyzine, itopride, metoclopramide, metopimazine, prochlorperazine, thiethylperazine, trimethobenzamide, cyclizine, dimenhydrinate, diphenhydramine, hydroxyzine, meclizine, promethazine, atropine, diphenhydramine, hyoscyamine, scopolamine, aprepitant, casopitant, ezlopitant, fosaprepitant, maropitant, netupitant, rolapitant, vestipitant, cerium oxalate, dexamethasone, lorazepam, midazolam, propofol, or a combination thereof.

In one embodiment, the present invention relates to the compound of formula (I) or the pharmaceutically acceptable salt thereof for use in the treatment or prevention of an inflammatory disease or an autoimmune disease. The present invention further relates to the pharmaceutical composition of the present invention comprising the compound of formula (I) or a pharmaceutically acceptable salt thereof for use in the treatment or prevention of an inflammatory disease or an autoimmune disease. Preferably, the said inflammatory disease or autoimmune disease is selected from ischemia, autoimmune bleeding disorder, septic shock, rheumatoid arthritis, inflammatory bowel disease, systemic lupus erythematosus (SLE), multiple sclerosis, asthma, osteoarthritis, osteoporosis, a fibrotic disease, dermatosis, psoriasis, atopic dermatitis, ultraviolet radiation (UV)-induced skin damage, psoriatic arthritis, ankylosing spondylitis, tissue and organ rejection, Alzheimer's disease, stroke, atherosclerosis, restenosis, diabetes, glomerulonephritis, Hodgkin's disease, cachexia, inflammation associated with infection, inflammation associated with viral infection, acquired immune deficiency syndrome (AIDS), adult respiratory distress syndrome, and ataxia telangiectasia.

In one embodiment, the present invention relates to the compound of formula (I) or the pharmaceutically acceptable salt thereof for use in the treatment or prevention of an infectious disease. The present invention further relates to the pharmaceutical composition of the present invention comprising the compound of formula (I) or a pharmaceutically acceptable salt thereof for use in the treatment or prevention of an infectious disease. Preferably, the said infectious disease is selected from a bacterial infectious disease, a protozoan infectious disease, a fungal infectious disease, and a helminth infectious disease.

The present invention further relates to the compound of formula (I) or the pharmaceutically acceptable salt thereof or the pharmaceutical composition comprising the compound of formula (I) or the pharmaceutically acceptable salt thereof for use in the treatment or prevention of an infectious disease caused by Gram-positive bacteria. Preferably, the said Gram-positive bacteria are selected from: *Listeria* spp., including e.g. *Listeria monocytogenes* or *Listeria welshimeri*; *Staphylococcus* spp., including e.g. *Staphylococcus aureus*, *Staphylococcus epidermidis, Staphylococcus saprophyticus*, *Staphylococcus lugdunensis*, *Staphylococcus schleiferi*, or *Staphylococcus caprae*; *Streptococcus* spp., including e.g. *Streptococcus pneumoniae*, *Streptococcus viridans*, *Streptococcus pyogenes*, or *Streptococcus agalactiae*; *Enterococcus* spp., including e.g. *Enterococcus faecalis* or *Enterococcus faecium*; *Bacillus* spp., including e.g. *Bacillus licheniformis*, *Bacillus subtilis*, *Bacillus anthracis*, *Bacillus cereus*, *Bacillus thuringiensis*, or *Bacillus larvae*; *Mycobacterium* spp., including e.g. *Mycobacterium tuberculosis*, *Mycobacterium bovis*, *Mycobacterium leprae, Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi, Mycobacterium fortuitum*, *Mycobacterium chelonei*, or *Mycobacterium marinum*; *Nocardia* spp., including e.g. *Nocardia asteroids*; and *Rhodococcus* spp., including e.g. *Rhodococcus equi.*

The present invention further relates to the compound of formula (I) or pharmaceutically acceptable salt thereof the pharmaceutical composition comprising the compound of formula (I) or the pharmaceutically acceptable salt thereof for use in the treatment or prevention of an infectious disease caused by Gram-negative bacteria. As defined herein, the said Gram-negative bacteria are preferably selected from: *Neisseria* spp., including e.g. *Neisseria gonorrhoeae* or *Neisseria meningitides*; *Moraxella* spp., including e.g. *Moraxella catarrhalis*; *Hemophilus* spp., including e.g. *Hemophilus influenzae*; *Klebsiella* spp., including e.g. *Klebsiella pneumoniae*; *Legionella spp.,* including e.g. *Legionella pneumophila*; *Pseudomonas* spp., including e.g. *Pseudomonas aeruginosa* or *Pseudomonas putida*; *Escherichia* spp., including e.g. *Escherichia coli*; *Proteus* spp., including e.g. *Proteus mirabilis*; *Enterobacter* spp., including e.g. *Enterobacter cloaceae*; *Serratia* spp., including e.g. *Serratia marcescens*; *Helicobacter* spp., including e.g. *Helicobacter pylori*; and *Salmonella* spp., including e.g. *Salmonella enteritidis* or *Salmonella typhi.*

The compound of formula (I) or a pharmaceutically acceptable salt thereof or the pharmaceutical composition of the invention can be combined with one or more anti-bacterial agents. In one embodiment, the said anti-bacterial agent is preferably effective against an infectious disease caused by Gram-positive bacteria. Preferably, the said Gram-positive bacteria are selected from: *Listeria* spp., including e.g. *Listeria monocytogenes* or *Listeria welshimeri; Staphylococcus* spp., including e.g. *Staphylococcus aureus*, *Staphylococcus epidermidis*, *Staphylococcus saprophyticus, Staphylococcus lugdunensis, Staphylococcus schleiferi,* or *Staphylococcus caprae*; *Streptococcus* spp., including e.g. *Streptococcus pneumoniae*, *Streptococcus viridans*, *Streptococcus pyogenes*, or *Streptococcus agalactiae; Enterococcus* spp., including e.g. *Enterococcus faecalis* or *Enterococcus faecium*; *Bacillus* spp., including e.g. *Bacillus licheniformis*, *Bacillus subtilis*, *Bacillus anthracis*, *Bacillus cereus*, *Bacillus thuringiensis*, or *Bacillus larvae*; *Mycobacterium* spp., including e.g. *Mycobacterium tuberculosis*, *Mycobacterium bovis*, *Mycobacterium leprae*, *Mycobacterium ulcerans, Mycobacterium kanasasii, Mycobacterium avium, Mycobacterium paratuberculosis, Mycobacterium scrofulaceam*, *Mycobacterium microti*, *Mycobacterium africanum*, *Mycobacterium canettii*, *Mycobacterium intracellulare, Mycobacterium simiae, Mycobacterium szulgai, Mycobacterium xenopi, Mycobacterium fortuitum*, *Mycobacterium chelonei*, or *Mycobacterium marinum*; *Nocardia* spp., including e.g. *Nocardia asteroids*; and *Rhodococcus* spp., including e.g. *Rhodococcus equi.* In one embodiment, the said anti-bacterial agent is preferably effective against an infectious disease caused by Gram-negative bacteria. The said Gram-negative bacteria are preferably selected from: *Neisseria* spp., including e.g. *Neisseria gonorrhoeae* or *Neisseria meningitides*; *Moraxella* spp., including e.g. *Moraxella catarrhalis*; *Hemophilus* spp., including e.g. *Hemophilus influenzae; Klebsiella* spp., including e.g. *Klebsiella pneumoniae*; *Legionella spp*., including e.g. *Legionella pneumophila*; *Pseudomonas* spp., including e.g. *Pseudomonas aeruginosa* or *Pseudomonas putida*; *Escherichia* spp., including e.g. *Escherichia coli*; *Proteus* spp., including e.g. *Proteus mirabilis*; *Enterobacter* spp., including e.g. *Enterobacter cloaceae*; *Serratia* spp., including e.g. *Serratia marcescens*; *Helicobacter* spp., including e.g. *Helicobacter pylori;* and *Salmonella* spp., including e.g. *Salmonella enteritidis* or *Salmonella typhi.*

The present invention further relates to the compound of formula (I) or the pharmaceutically acceptable salt thereof or the pharmaceutical composition comprising the compound of formula (I) or the pharmaceutically acceptable salt thereof for use in the treatment or prevention of a protozoan infectious disease. Preferably, the said protozoan infectious disease is caused by a protozoan of the phylum Apicomplexa. More preferably, the said protozoan infectious disease is caused by a protozoan selected from: *Plasmodium* spp., including e.g. *Plasmodium falciparum*, *Plasmodium malariae*, *Plasmodium vivax*, *Plasmodium ovale*, or *Plasmodium knowlesi*; *Leishmania* spp., including e.g. *Leishmania major*, *Leishmania tropica*, *Leishmania aethiopica*, *Leishmania mexicana*, *Leishmania braziliensis*, *Leishmania donovani*, *Leishmania infantum*, or *Leishmania chagasi*; *Trypanosoma* spp., including e.g. *Trypanosoma brucei, Trypanosoma cruzi, Trypanosoma simiae, Trypanosoma avium, Trypanosoma congolense, Trypanosoma equinum*, *Trypanosoma equiperdum*, *Trypanosoma evansi*, or *Trypanosoma suis*; *Babesia* spp., including e.g. *Babesia microti* or *Babesia bigemina*; and *Toxoplasma* spp., including e.g. *Toxoplasma gondii.*

The compound of formula (I) or a pharmaceutically acceptable salt thereof, or the pharmaceutical composition comprising the same of the present invention can be used in combination with an antiprotozoan agent. Preferably, the antiprotozoan agent to be used in combination with the compound of the invention or the pharmaceutical composition of the invention is useful in the treatment and/or prevention of protozoan infectious disease is caused by a protozoan selected from: *Plasmodium* spp., including e.g. *Plasmodium falciparum*, *Plasmodium malariae*, *Plasmodium vivax*, *Plasmodium ovale*, or *Plasmodium knowlesi*; *Leishmania* spp., including e.g. *Leishmania major*, *Leishmania tropica, Leishmania aethiopica, Leishmania mexicana, Leishmania braziliensis, Leishmania donovani, Leishmania infantum*, or *Leishmania chagasi*; *Trypanosoma* spp., including e.g. *Trypanosoma brucei*, *Trypanosoma cruzi, Trypanosoma simiae, Trypanosoma avium, Trypanosoma congolense, Trypanosoma equinum, Trypanosoma equiperdum*, *Trypanosoma evansi*, or *Trypanosoma suis*; *Babesia* spp., including e.g. *Babesia microti* or *Babesia bigemina*; and *Toxoplasma* spp., including e.g. *Toxoplasma gondii.*

Thus, the present invention further relates to the compound of formula (I) or a pharmaceutically acceptable salt thereof, or the pharmaceutical composition comprising the same for use in combination with an antiprotozoan agent, wherein the antiprotozoan agent is preferably selected from acranil, tinidazole, ipronidazole, ethylstibamine, pentamidine, acetarsone, aminitrozole, anisomycin, nifuratel, tinidazole, benzidazole, and suramin.

The present invention further relates to the compound of formula (I) or the pharmaceutically acceptable salt thereof or the pharmaceutical composition comprising the compound of formula (I) or the pharmaceutically acceptable salt thereof for use in the treatment or prevention of a fungal infectious disease. Preferably, the said fungal infectious disease is caused by a fungus selected from: *Aspergillus* spp., including e.g. *Aspergillus fumigatus, Aspergillus flavus,* or *Aspergillus clavatus*; *Candida* spp., including e.g. *Candida albicans*, *Candida stellatoidea*, *Candida tropicalis*, *Candida pseudotropicalis*, *Candida krusei*, *Candida parapsilosis*, or *Candida guilliermondii*; *Cryptococcus* spp., including e.g. *Cryptococcus neoformans*, *Cryptococcus laurentii*, *Cryptococcus albidus*, or *Cryptococcus gattii*; *Histoplasma* spp., including e.g. *Histoplasma capsulatum*; *Pneumocystis* spp., including e.g. *Pneumocystis jirovecii* or *Pneumocystis carinii*; *Stachybotrys* spp., including e.g. *Stachybotrys chartarum*; *Basidiobolus* spp., including e.g. *Basidiobolus ranarum*; *Blastomyces* spp., including e.g. *Blastomyces dermatitidis*; *Coccidioides* spp., including e.g. *Coccidioides immitis* or *Coccidioides posadasii*; *Conidiobolus* spp., including e.g. *Conidiobolus coronatus* or *Conidiobolus incongruous*; and *Madurella* spp., including e.g. *Madurella mycetomatis* or *Madurella grisea.*

The compounds of the formula (I) can be used in combination with one or more other medicaments. Preferably the other medicament will be a further medicament which is useful in treating, ameloriating or preventing a fungal infection, more preferably a further medicament which is useful in treating, ameloriating or preventing a fungal infection that is caused by a fungus selected from *Aspergillus* spp., including e.g. *Aspergillus fumigatus*, *Aspergillus flavus*, or *Aspergillus clavatus*; *Candida* spp., including e.g. *Candida albicans*, *Candida stellatoidea*, *Candida tropicalis*, *Candida pseudotropicalis*, *Candida krusei*, *Candida parapsilosis*, or *Candida guilliermondii*; *Cryptococcus* spp., including e.g. *Cryptococcus neoformans*, *Cryptococcus laurentii*, *Cryptococcus albidus*, or *Cryptococcus gattii*; *Histoplasma* spp., including e.g. *Histoplasma capsulatum*; *Pneumocystis* spp., including e.g. *Pneumocystis jirovecii* or *Pneumocystis carinii*; *Stachybotrys* spp., including e.g. *Stachybotrys chartarum*; *Basidiobolus* spp., including e.g. *Basidiobolus ranarum*; *Blastomyces* spp., including e.g. *Blastomyces dermatitidis*; *Coccidioides* spp., including e.g. *Coccidioides immitis* or *Coccidioides posadasii*; *Conidiobolus* spp., including e.g. *Conidiobolus coronatus* or *Conidiobolus incongruous*; and *Madurella* spp., including e.g. *Madurella mycetomatis* or *Madurella grisea.*

Thus, the present invention relates to the compound of formula (I) or a pharmaceutically acceptable salt thereof, or the pharmaceutical composition of the present invention comprising the compound of formula (I) or a pharmaceutically acceptable salt thereof and a pharmaceutically acceptable carrier, for use in combination with an antifungal agent.

The compound of the formula (I), the pharmaceutically acceptable salt thereof or the pharmaceutical composition comprising the same can be used in combination with one or more antifungal agents capable of at least one of: a) inhibiting ergosterol biosynthesis; b) binding to ergosterol; c) inhibiting 1,3-P-glucan synthase; d) inhibiting epoxidase; e) inhibiting leucyl-tRNA synthetase; and/or f) inhibition of elongation factor 2. Thus, the additional antifungal agent may be for example be selected from the group consisting of polyene antifungal agents (such as Natamycin, Rimocidin, Filipin, Nystatin, Amphotericin B or Candicin), azole antifungal agents (such as miconazole, ketoconazole, clotromazole, econazole, bifonazole, butoconazole, fenticonazole, isoconazole, oxiconazole, seraconazole, sulconazole, tioconazole, fluconazole, itraconazole, isavuconazole, ravuconazole, posaconazole, voriconazole terconazole, or abafungin), allylamine antifungal agents (such as Terbinafme, Amorolfine, Naftifme or Butenafme) and echinocandins (such as Anidulafungin, Caspofungin or Micafungin).

The compound of formula (I), the pharmaceutically acceptable salt thereof or the pharmaceutical composition comprising the same may also be useful in treating and/or preventing a fungal infection disease, wherein the said fungal infectious disease cannot be treated in a curable manner by any of the antifungal agents capable of at least one of a) inhibiting ergosterol biosynthesis; b) binding to ergosterol; c) inhibiting 1,3-P-glucan synthase; d) inhibiting epoxidase; e) inhibiting leucyl-tRNA synthetase; and/or f) inhibition of elongation factor 2. Thus, the compound of formula (I) of the present invention or the pharmaceutical composition comprising the same may also be useful in treating and/or preventing fungal infectious disease that is resistant to any of the agents selected from the group consisting of polyene antifungal agents (such as Natamycin, Rimocidin, Filipin, Nystatin, Amphotericin B or Candicin), azole antifungal agents (such as miconazole, ketoconazole, clotromazole, econazole, bifonazole, butoconazole, fenticonazole, isoconazole, oxiconazole, seraconazole, sulconazole, tioconazole, fluconazole, itraconazole, isavuconazole, ravuconazole, posaconazole, voriconazole terconazole, or abafungin), allylamine antifungal agents (such as Terbinafme, Amorolfine, Naftifme or Butenafme) and echinocandins (such as Anidulafungin, Caspofungin or Micafungin).

The present invention further relates to the compound of formula (I) or the pharmaceutically acceptable salt thereof or the pharmaceutical composition comprising the compound of formula (I) or the pharmaceutically acceptable salt thereof as defined herein for use in the treatment or prevention of a helminth infectious disease. Preferably, the said helminth infectious disease is caused by a roundworm, a tapeworm, a trematode, or an acanthocephalan. Further preferably, the said helminth infectious disease is caused by a helminth selected from: *Wuchereria* spp., including e.g. *Wuchereria bancrofti*; *Brugia* spp., including e.g. *Brugia malayi*; *Onchocerca* spp., including e.g. *Onchocerca volvulus; Ascaris* spp., including e.g. *Ascaris lumbricoides*; *Trichuris* spp., including e.g. *Trichuris trichiura, Trichuris campanula*, *Trichuris serrata*, *Trichuris suis*, *Trichuris muris*, or *Trichuris vulpis*; *Necatorspp.,* including e.g. *Necator* *americanus; Ancylostoma* spp., including e.g. *Ancylostoma duodenale*; *Trichostrongylus* spp., including e.g. *Trichostrongylus affinis*, *Trichostrongylus sigmodontis*, *Trichostrongylus tenuis*, or *Trichostrongylus retortaeformis*; *Dracunculus* spp., including e.g. *Dracunculus medinensis*; *Baylisascaris* spp., including e.g. *Baylisascaris procyonis, Baylisascaris melis*, *Baylisascaris transfuga*, *Baylisascaris columnaris*, *Baylisascaris devosi*, *Baylisascaris laevis*, *Baylisascaris shroederi*, or *Baylisascaris potosis*; *Echinococcus* spp., including e.g. *Echinococcus granulosus*, *Echinococcus multilocularis*, *Echinococcus vogeli*, or *Echinococcus oligarthrus*; *Hymenolepis* spp., including e.g. *Hymenolepis diminuta, Hymenolepis nana, Hymenolepis apodemi, Hymenolepis asymetrica, Hymenolepis horrida, Hymenolepis rymzhanovi*, or *Hymenolepis microstoma*; *Taenia* spp., including e.g. *Taenia multiceps*, *Taenia glomerata, Taenia brauni, Taenia asiatica, Taenia bubesei, Taenia crassiceps, Taenia hydatigena, Taenia mustelae, Taenia ovis*, *Taenia pisiformis*, *Taenia rileyi*, *Taenia saginata*, *Taenia solium*, *Taenia taeniaeformis*, or *Taenia serialis; Gastrodiscoidesspp.,* including e.g. *Gastrodiscoides hominis* or *Gastrodiscoides discoides*; *Watsonius* spp., including e.g. *Watsonius watsoni*; *Clonorchis* spp., including e.g. *Clonorchis sinensis*; *Fasciola* spp., including e.g. *Fasciola hepatica* or *Fasciola gigantica*; *Fasciolopsis* spp., including e.g. *Fasciolopsis buski*; *Opisthorchis* spp., including e.g. *Opisthorchis chabaudi*, *Opisthorchis felineus*, *Opisthorchis gomtii*, *Opisthorchis parasiluri*, or *Opisthorchis viverrini*; *Paragonimus* spp., including e.g. *Paragonimus westermani*, *Paragonimus africanus*, *Paragonimus caliensis*, *Paragonimus compactus, Paragonimus ecuadoriensis, Paragonimus heterotremus, Paragonimus hueitugensis, Paragonimus iloktsuenensis*, *Paragonimus kellicotti*, *Paragonimus mexicanus*, *Paragonimus miyazakii*, *Paragonimus ohirai, Paragonimus pulmonalis*, *Paragonimus peruvianus*, *Paragonimus sadoensis*, *Paragonimus skrjabini*, or *Paragonimus uterobilateralis*; *Schistosoma* spp., including e.g. *Schistosoma bomfordi*, *Schistosoma bovis*, *Schistosoma curassoni, Schistosoma datta, Schistosoma edwardiense, Schistosoma guineensis, Schistosoma haematobium, Schistosoma harinasutai, Schistosoma hippopotami, Schistosoma incognitum, Schistosoma indicum, Schistosoma intercalatum, Schistosoma japonicum, Schistosoma kisumuensis, Schistosoma leiperi, Schistosoma malayensis, Schistosoma mansoni, Schistosoma margrebowiei, Schistosoma mattheei, Schistosoma mekongi, Schistosoma ovuncatum*, *Schistosoma nasale*, *Schistosoma rodhaini*, *Schistosoma sinensium*, *Schistosoma spindale*, or *Schistosoma turkestanicum*; *Moniliformis* spp., including e.g. *Moniliformis moniliformis*; *Toxocara* spp., including e.g. *Toxocara canis* or *Toxocara cati*; *Enterobius* spp., including e.g. *Enterobius vermicularis* or *Enterobius gregorii*; and *Strongyloides* spp., including e.g. *Strongyloides stercoralis*, *Strongyloides fuelleborni*, or *Strongyloides kellyi.*

The present invention further relates to the compound of formula (I) or a pharmaceutically acceptable salt thereof, or the pharmaceutical composition comprising the same of the present invention, for use in combination with an anthelmintic drug. Examples of such anthelmintic agents include ivermectin, avermectin, abamectin, emamectin, epilinomectin, doramectin, macrocyclic lactone antihelmintic compounds such as selamectin, moxidectin, nemadectin and milbemycin, semi-synthetic and biosynthetic avermectin / milbemycin derivatives such as those described in US Pat. No. 5,015,630, WO9415944, and WO9522552, benzimidazoles such as albendazole, cambendazole, fenbendazole, flubendazole, mebendazole, oxyfendazole, oxybendazole, and parbendazole, imidazothiazole and tetrahydropyrimidines such as tetramisol, levamisole, pyrantel pamoate, oxantel and morantel, fluoxides such as triclabendazole and chlorthrone, and cestosides such as praziquantel and epsiprantel. Thus, the present invention relates to the compound of formula (I) or a pharmaceutically acceptable salt thereof, or the pharmaceutical composition comprising the same of the present invention, for use in combination with an anthelmintic drug selected from ivermectin, avermectin, abamectin, emamectin, epilinomectin, doramectin, selamectin, moxidectin, nemadectin milbemycin, albendazole, cambendazole, fenbendazole, flubendazole, mebendazole, oxyfendazole, oxybendazole, parbendazole, tetramisol, levamisole, pyrantel pamoate, oxantel morantel, triclabendazole chlorthrone, praziquantel and epsiprantel.

Preferably, the anthelmintic drug(s) to be used in combination with the compound of formula (I) or a pharmaceutically acceptable salt thereof, or in combination with the pharmaceutical composition of the present invention, are useful in the treatment or prevention of a helminth infectious disease. Preferably, the said helminth infectious disease is caused by a roundworm, a tapeworm, a trematode, or an acanthocephalan. Further preferably, the said helminth infectious disease is caused by a helminth selected from: *Wuchereria* spp., including e.g. *Wuchereria bancrofti*; *Brugia* spp., including e.g. *Brugia malayi*; *Onchocerca* spp., including e.g. *Onchocerca volvulus*; *Ascaris* spp., including e.g. *Ascaris lumbricoides*; *Trichuris* spp., including e.g. *Trichuris trichiura*, *Trichuris campanula*, *Trichuris serrata*, *Trichuris suis*, *Trichuris muris*, or *Trichuris vulpis*; *Necatorspp*., including e.g. *Necator americanus*; *Ancylostoma* spp., including e.g. *Ancylostoma duodenale*; *Trichostrongylus* spp., including e.g. *Trichostrongylus affinis*, *Trichostrongylus sigmodontis*, *Trichostrongylus tenuis*, or *Trichostrongylus retortaeformis*; *Dracunculus* spp., including e.g. *Dracunculus medinensis*; *Baylisascaris* spp., including e.g. *Baylisascaris procyonis*, *Baylisascaris melis*, *Baylisascaris transfuga*, *Baylisascaris columnaris*, *Baylisascaris devosi*, *Baylisascaris laevis, Baylisascaris shroederi,* or *Baylisascaris potosis*; *Echinococcus* spp., including e.g. *Echinococcus granulosus, Echinococcus multilocularis*, *Echinococcus vogeli*, or *Echinococcus oligarthrus*; *Hymenolepis* spp., including e.g. *Hymenolepis diminuta*, *Hymenolepis nana*, *Hymenolepis apodemi*, *Hymenolepis asymetrica*, *Hymenolepis horrida*, *Hymenolepis rymzhanovi*, or *Hymenolepis microstoma*; *Taenia* spp., including e.g. *Taenia multiceps*, *Taenia glomerata*, *Taenia brauni*, *Taenia asiatica*, *Taenia bubesei*, *Taenia crassiceps*, *Taenia hydatigena*, *Taenia mustelae*, *Taenia ovis*, *Taenia pisiformis*, *Taenia rileyi, Taenia saginata*, *Taenia solium*, *Taenia taeniaeformis*, or *Taenia serialis; Gastrodiscoides* spp., including e.g. *Gastrodiscoides hominis* or *Gastrodiscoides discoides*; *Watsonius* spp., including e.g. *Watsonius watsoni*; *Clonorchis* spp., including e.g. *Clonorchis sinensis*; *Fasciola* spp., including e.g. *Fasciola hepatica* or *Fasciola gigantica*; *Fasciolopsis* spp., including e.g. *Fasciolopsis buski*; *Opisthorchis* spp., including e.g. *Opisthorchis chabaudi, Opisthorchis felineus, Opisthorchis gomtii, Opisthorchis parasiluri,* or *Opisthorchis viverrini*; *Paragonimus* spp., including e.g. *Paragonimus westermani*, *Paragonimus africanus*, *Paragonimus caliensis*, *Paragonimus compactus*, *Paragonimus ecuadoriensis, Paragonimus heterotremus, Paragonimus hueitugensis, Paragonimus iloktsuenensis*, *Paragonimus kellicotti, Paragonimus mexicanus, Paragonimus miyazakii, Paragonimus ohirai, Paragonimus pulmonalis, Paragonimus peruvianus*, *Paragonimus sadoensis, Paragonimus skrjabini,* or *Paragonimus uterobilateralis; Schistosoma* spp., including e.g. *Schistosoma bomfordi, Schistosoma bovis, Schistosoma curassoni, Schistosoma datta, Schistosoma edwardiense, Schistosoma guineensis, Schistosoma haematobium, Schistosoma harinasutai, Schistosoma hippopotami, Schistosoma incognitum, Schistosoma indicum, Schistosoma intercalatum, Schistosoma japonicum, Schistosoma kisumuensis, Schistosoma leiperi, Schistosoma malayensis, Schistosoma mansoni, Schistosoma margrebowiei, Schistosoma mattheei, Schistosoma mekongi, Schistosoma ovuncatum, Schistosoma nasale*, *Schistosoma rodhaini, Schistosoma sinensium, Schistosoma spindale,* or *Schistosoma turkestanicum*; *Moniliformis* spp., including e.g. *Moniliformis moniliformis; Toxocara* spp., including e.g. *Toxocara canis* or *Toxocara cati*; *Enterobius* spp., including e.g. *Enterobius vermicularis* or *Enterobius gregorii*; and *Strongyloides* spp., including e.g. *Strongyloides stercoralis, Strongyloides fuelleborni,* or *Strongyloides kellyi.*

In certain helminth infectious diseases, a surgery may be required, for example in the case of intestinal obstruction or removal of helminths from the biliary tree. In the cases where the surgery is not performed as an emergency surgery and the subject can be properly prepared therefor, the subject may be pretreated with an anthelmintic drug. Therefore, the present invention further relates to the compound of formula (I) or a pharmaceutically acceptable salt thereof, or the pharmaceutical composition comprising the same of the present invention, for use in pretreatment of a subject before a surgical removal of helminths, preferably wherein the surgical removal of helminths is a surgical removal of helminths from the biliary tree.

The subject or patient to be treated in accordance with the present invention may be an animal (e.g., a non-human animal). Preferably, the subject/patient is a mammal. More preferably, the subject/patient is a human (e.g., a male human or a female human) or a non-human mammal (such as, e.g., a guinea pig, a hamster, a rat, a mouse, a rabbit, a dog, a cat, a horse, a monkey, an ape, a marmoset, a baboon, a gorilla, a chimpanzee, an orangutan, a gibbon, a sheep, cattle, or a pig). Most preferably, the subject/patient to be treated in accordance with the invention is a human.

The term "treatment" of a disorder or disease, as used herein, is well known in the art. "Treatment" of a disorder or disease implies that a disorder or disease is suspected or has been diagnosed in a patient/subject. A patient/subject suspected of suffering from a disorder or disease typically shows specific clinical and/or pathological symptoms which a skilled person can easily attribute to a specific pathological condition (i.e., diagnose a disorder or disease).

The "treatment" of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment (such as the exemplary responses as described herein above). The treatment of a disorder or disease may, *inter alia,* comprise curative treatment (preferably leading to a complete response and eventually to healing of the disorder or disease) and palliative treatment (including symptomatic relief).

The term "prevention" of a disorder or disease, as used herein, is also well known in the art. For example, a patient/subject suspected of being prone to suffer from a disorder or disease may particularly benefit from a prevention of the disorder or disease. The subject/patient may have a susceptibility or predisposition for a disorder or disease, including but not limited to hereditary predisposition. Such a predisposition can be determined by standard methods or assays, using, e.g., genetic markers or phenotypic indicators. It is to be understood that a disorder or disease to be prevented in accordance with the present invention has not been diagnosed or cannot be diagnosed in the patient/subject (for example, the patient/subject does not show any clinical or pathological symptoms). Thus, the term "prevention" comprises the use of a compound of the present invention before any clinical and/or pathological symptoms are diagnosed or determined or can be diagnosed or determined by the attending physician.

In another embodiment, the present invention relates to a plant protection composition comprising a compound of formula (I) or an agriculturally acceptable salt thereof.

Agriculturally acceptable salt of the compound of formula (I) can be formed e.g. by reacting the compound with an acid of the anion in question if the compound of formula I has a basic functionality or by reacting an acidic compound of formula I with a suitable base. Suitable agriculturally acceptable salts are especially the salts of those cations or the acid addition salts of those acids whose cations and anions, which are known and accepted in the art for the formation of salts for agricultural or veterinary use respectively, and do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH⁴⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or -CH₂-phenyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethy|ammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts that are agriculturally acceptable are preferably chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting a compound of formula (I) with an acid of the corresponding anion, preferably of hydrochloric acid, hydrobromic acid, sulfuric acid, phosphoric acid or nitric acid.

In a further embodiment, the present invention relates to a method of controlling a plant disease. The method of controlling a plant disease of the present invention as defined herein comprises applying a compound of formula (I) as defined herein or an agriculturally acceptable salt thereof or the plant protection composition of the present invention.

The compound or the plant protection composition is applied by treating a plant or a part thereof with the compound or the composition. In certain embodiments of the method of controlling a plant disease of the present invention, the compound or the plant protection composition is applied by treating a plant seed or bulb with the compound or the composition. For example, the compound or the plant protection composition can be formulated as wettable powders, emulsifiable concentrates, dusts, granular formulations, aerosols, or flowable emulsion concentrates. In such formulations, the the compound or the plant protection composition may be extended with a liquid or solid carrier and, when desired, suitable surfactants may be incorporated.

The compound or the plant protection composition is applied by treating the soil on which a plant is cultivated with the compound or the composition. Soil to be planted with vegetables, shrubs, or trees can be treated with the compound or the plant protection composition of the invention preferably for control of a variety of plant pathogens. Treatment is preferably done several days or weeks before planting. The compound or the plant protection composition of the invention can be applied by either a mechanized route, e.g., a tractor, or with hand applications.

The compound or the plant protection composition can be applied by hydroponic treatment of a plant with the compound or the composition.

Thus, the method of controlling a plant disease of the present invention relates to an embodiment, wherein the compound or the plant protection composition is applied by treating a plant or a part thereof with the compound or the composition, by treating the soil on which a plant is cultivated with the compound or the composition, by hydroponic treatment of a plant with the compound or the composition, or by treating a plant seed or bulb with the compound or the composition.

A plant disease as referred to herein is not meant to be particularly limited. In certain embodiments, the plant disease is caused by a plant pathogenic organism.

The plant pathogenic organism can be a bacterium. Preferably, the said bacterium is selected from the genera of *Erwinia, Pectobacterium, Pantoea, Agrobacterium, Pseudomonas, Ralstonia, Burkholderia, Acidovorax, Xanthomonas, Clavibacter, Streptomyces, Xylella, Spiroplasma,* and *Phytoplasma.*

The plant pathogenic organism can be a protozoan, for example a protozoan selected from *Phytomonas. Phytomonas* is a genus of trypanosomatids that infect plant species. Examples include *Phytomonas serpens* in tomato, P. *staheli* in coconut and oil palm, and *P. leptovasorum* in coffee.

The plant pathogenic organism can be a fungus. Preferably, the said plant pathogenic fungus may be selected from the group consisting of *Albugo* spp., *Alternaria* spp., *Anisogramma anomala, Apiosporina morbosa, Ascochyta* spp., *Aureobasidium zeae, Bipolaris* spp., *Blumeria* spp., *Blumeriella jaapii, Botritis* spp., *Botryosphaeria dothidea, Ceratocystic paradoxa, Cercospora* spp., *Cercosporidium* spp., *Cladosporium* spp., *Cochiliobolus* spp., *Colletotrichum* spp., *Corynespora cassiicola, Cristulariella moricola, Diaporthe phaseolorum, Didymella bryoniae, Drechslera tritici, Entyloma oryzai, Erysiphe* spp., *Fluvia fluva, Fusarium* spp., *Gaeumannomyces graminis, Gnomonia* spp., *Gremmeniella abietina, Helminthosporium* spp., *Leptosphaerulina crassiasca, Leveillula taurica, Lophodermium hypophyllum, Macrophomina phaseoli, Magnaporthe* spp., *Microdochium* spp., *Microsphaera* spp., *Monilinia* spp., *Mycosphaerella* spp., *Myrothedium roridum*, *Oidiopsis sicula*, *Passalora puncta*, *Penicillium* spp., *Peronospora* spp., *Phaeocryptopus gaeumannii*, *Phakopsora* spp., *Phoma arachidicola*, *Phragmidium potentillae, Phytophtora* spp., *Plasmopara* spp., *Plectosporium tabacinum*, *Pleospora herbarum*, *Podosphaera* spp., *Pseudocercosporella* spp., *Pseudoperonospora cubensis*, *Puccinia* spp., *Pucciniastrum vaccinii*, *Pyrenophora* spp., *Pythium* spp., *Ramularia cynarae*, *Rhizoctonia* spp., *Rhizosphaera* spp., *Rhynchosporium secalis*, *Sclerotinia* spp., *Sclerotium* spp., *Selenophoma* spp., *Septoria* spp., *Setosphaeria turcica*, *Sirococcus conigenus*, *Sphaerotheca* spp., *Stagonospora nordorum*, *Stemphyllium botryosum*, *Taphrina deformans*, *Thielaviopsis* spp., *Tilletia barclayana*, *Tranzschelia discolor*, *Uncinula necator*, *Uromyces appendiculatus*, *Ustilaginoidea virens*, *Ustilago* spp., *Venturia* spp., *Verticillium* spp. and *Wilsonomyces carpophilus.*

The plant pathogenic organism can be an oomycete. Preferably, the oomycete in the method of the present invention is selected from *Phytophthora.* Further preferably, the oomycete (also referred to as oomycete plant pathogen) is selected from *Phytophthora infestans*, *Phytophthora ipomoeae*, *Phytophthora mirabilis*, *Phytophthora phaseoli*, *Phytophthora capsici, Phytophthora porri, Phytophthora parasitica, Hyaloperonospora arabidopsidis, Peronospora farinosa, Pseudoperonospora cubensis, Hyaloperonospora parasitica, Peronospora destructor, Bremia lactucae, Pseudoperonospora cubensis, Pseudoperonospora humuli, Peronospora destructor, Albugo Candida, Albugo occidentalis*, and *Pythium spp.*

The plant pathogenic organism can be a nematode. Nematodes are small, worm-like animals frequently associated with a variety of destructive diseases in plants, Pathogenic nematodes are a major agricultural problem, causing significant crop and yield losses worldwide. The potato cyst nematodes *Globodera rostochiensis* and *Globodera pallida*, for example, are key pests of the potato, while the beet cyst nematode *Heterodera schachtii* is a major problem for sugar beet growers in Europe and the United States. Thus, preferably the nematode is selected from Heterodera spp, *Meloidogyne spp*., *Globodera spp. Heterodera glycines* (soybean cyst nematode); *Heterodera schachtii* (beet cyst nematode); *Heterodera avenae* (cereal cyst nematode); *Globodera rostochiensis* and *Globodera pailida.*

In one embodiment, the plant pathogenic organism is an insect, preferably a hemipteran insect selected from aphids, whiteflies and psyllids.

In one embodiment of the present invention, the plant pathogenic organism is selected from a bacterium, a protozoan, a fungus, an oomycete, a nematode, and an insect.

The plant to be treated is not particularly limited. Preferably however, within the scope of the present invention the plant is a crop plant. Furthermore, the said crop plant is preferably selected from rice, wheat, corn/maize, sugarcane, potatoes, manioc, sorghum, millets, peanuts, beans, sweet potatoes, barleys, bananas, soybeans, tomatoes, grapes, apples, onions, cucumbers, garlic, melons, mangos, spinach, strawberries, peaches, nectarines, lettuce, chicory, peas, coffee, tea, tobacco, and cotton.

### Non-therapeutic use

The present invention further encompasses non-therapeutic uses of the compound of formula (I) of the present invention. As it is to be understood herein, the reference to the compound of formula (I) also encompasses its pharmaceutically acceptable or agriculturally acceptable salt, as defined hereinabove.

In one embodiment, the present invention relates to a disinfectant comprising a compound of formula (I).

The disinfectant of the present invention is preferably an aqueous disinfectant formulation or an alcohol-based disinfectant formulation, preferably an ethanol-based disinfectant formulation. In certain embodiments of the present invention, the disinfectant of the present invention further comprises a surfactant. The surfactant allows for the creation of a "foaming effect" when the disinfectant solution is applied to a surface to be treated. The creation of a foam allows for the disinfectant solutions to remain in contact with the surface to be treated for longer periods of time.

The aqueous disinfectant formulation of the present invention in certain embodiments is capable of generating a foam when applied to a surface to be disinfected. The foam adheres to the surface to be disinfected for a time sufficient to ensure eradication of the non-indigenous and/or pathogenic bacterial, microbial, fungal and/or viral population.

The disinfectant formulations of the present invention may be applied onto a surface to be disinfected (i.e. cleaned) by means of a variety of spraying techniques. In one embodiment, the disinfectant of the present invention are applied using a diffuser or a mist blower. Alternatively, the disinfectant formulations of the present invention can also be formulated into aerosol formulations. In certain embodiments, the disinfectant of the present invention may be applied to the surface to be disinfected by using a foamer.

The disinfectant of the present invention preferably exhibits antibacterial properties, antifungal properties and/or antiviral properties. More preferably, the disinfectant of the present invention exhibits antibacterial properties and/or antifungal properties.

Further accordingly, in one embodiment the present invention relates to non-therapeutic use of a compound of formula (I) of the present invention or the disinfectant of the present invention for disinfecting or sterilizing an inanimate object.

In another embodiment, the present invention relates to a method of disinfecting or sterilizing an inanimate object. The said method of the present invention comprises applying a compound of formula (I) as defined hereinabove or the disinfectant of the present invention to said inanimate object. The inanimate object is not particularly limited within the scope of the present invention and any object could in principle be disinfected according to the instant method. In certain embodiments, the said inanimate object may also be referred to as a surface to be disinfected. Preferably, in certain embodiments the inanimate object is a technical device, preferably wherein the technical device is selected from a catheter, a medical implant, a surgical instrument, contact lenses, and a food-processing device.

In one embodiment of the present invention, the compound of formula (I) can be used in vitro as a proteasome inhibitor. Thus, the present invention relates to *In vitro* use of a compound of formula (I) as a proteasome inhibitor.

The ubiquitin-proteasome system responsible for degrading misfolded and malfunctioning proteins in eukaryotes plays an essential role in cell-cycle regulation and apoptosis. The system is also involved in degrading repressors of the insect immune response cascade. The proteasome 20S core particle, the catalytic core of the system, is assembled from four stacked heptameric rings adopting an α₁₋₇β₁₋₇β₁₋₇α₁₋₇ stoichiometry. The active site nucleophile of each proteolytic center is an N-terminal threonine (Thr1) located at subunits β1 (caspase-like activity), β2 (trypsin-like activity), and β5 (chymotrypsin-like activity). Natural products featuring a β-lactone moiety, such as omuralide, belactosins, and cystargolides, have been proven to suppress the proteolytic activity of the core particle (Groll, M., Larionov, O. V., Huber, R. & de Meijere, A. Inhibitor-binding mode of homobelactosin C to proteasomes: New insights into class I MHC ligand generation. Proc. Natl. Acad. Sci. U. S. A. 103, 4576-4579 (2006)). Their uniform mode of proteasome inhibition relies on opening of the β-lactone and transesterification upon nucleophilic attack by the catalytic N-terminal threonine (Thr1Oγ) (Michael, G. & Barbara, C. P. Proteasome Structure, Function, and Lessons Learned from Beta-Lactone Inhibitors. Curr. Top. Med. Chem. 11, 2850-2878 (2011); M. Groll, R. Huber, B. C. Potts, Crystal Structures of Salinosporamide A (NPI-0052) and B (NPI-0047) in Complex with the 20S Proteasome Reveal Important Consequences of β-Lactone Ring Opening and a Mechanism for Irreversible Binding, J. Am. Chem. Soc. 128, 5136 - 5141 (2006)).

Without being bound by theory, it has been found that the compounds of the present invention are capable of inhibiting proteasome. In particular, the present invention provides compounds which are suitable for use as pan-proteasome inhibitors, i.e. are capable of inhibiting all subunits of the eukaryotic proteasome (i.e., the β1, β2 and β5 subunits of the eukaryotic proteasome). The compounds of the present invention are furthermore suitable for use as selective inhibitors of proteasomal subunits. Preferably, the compounds of the present invention are capable of inhibiting the β5 subunit of the eukaryotic proteasome (which is responsible for chymotrypsin activity of the proteasome) selectively over the β1 and β2 subunits. The compounds of the present invention are further suitable for inhibiting the subunits of the core particle of the immunoproteasome (i.e., the iβ1, iβ2 and iβ5 subunits). Furthermore, it is considered that the compound 1 is an XPuniversal virulence factor against insects, as well as soil-living food competitors like protozoa, through disturbing the ubiquitin-proteasome system and thereby causing cell-cycle disturbance and immunodeficiency. The compounds of the present invention are furthermore suitable for therapeutic applications that require the active agent to cross the blood-brain barrier, for example, for the treatment of glioma (B. Potts et al., Marizomib, a proteasome inhibitor for all seasons: preclinical profile and a framework for clinical trials, Curr Cancer Drug Targets, 11(3), 254-84 (2011)).

Furthermore, without being bound by theory, the compounds of the present invention have been shown to be covalent inhibitors of the proteasome. Their mechanism of inhibition is considered to rely on opening of the β-lactone and transesterification upon nucleophilic attack by the catalytic N-terminal threonine (Thr1Oγ). Moreover, the compounds of the invention are suitable for use as reversible inhibitors of the proteasome, i.e. the catalytically active Thr1Oγ can be restored by slow hydrolysis of the acyl-enzyme adduct. In certain cases, β-lactone inhibitors can be modified to prevent the slow hydrolysis of the formed acyl-enzyme adduct (M. Groll, R. Huber, B. C. Potts, Crystal Structures of Salinosporamide A (NPI-0052) and B (NPI-0047) in Complex with the 20S Proteasome Reveal Important Consequences of β-Lactone Ring Opening and a Mechanism for Irreversible Binding, J. Am. Chem. Soc. 128, 5136-5141 (2006); M. Groll, K.A. McArthur, V.R. Macherla, R.R. Manam, and B.C. Potts, Snapshots of the Fluorosalinosporamide/20S Complex Offer Mechanistic Insights for Fine Tuning Proteasome Inhibition, J. Med. Chem. 52, 17, 5420-5428 (2009)).

In accordance with the above, the present invention particularly relates to the following items:
1. A compound of formula (I) 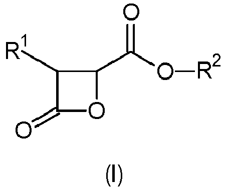 wherein:
   R¹ is selected from hydrogen, C₁₋₈ alkyl, C₂₋₈ alkenyl, C₂₋₈ alkynyl, -(C₁₋₆ alkylene)-O-R¹¹, -(C₁₋₆ alkylene)-S-R¹¹, -(C₁₋₆ alkylene)-N(R¹¹)-R¹¹, -(C₁₋₆ alkylene)-CO-R¹¹, -(C₁₋₆ alkylene)-COO-R¹¹, -(C₁₋₆ alkylene)-O-CO-(C₁₋₆ alkyl), -(C₁₋₆ alkylene)-CO-N(R¹¹)-R¹¹, -(C₁₋₆ alkylene)-N(R¹¹)-CO-(C₁₋₆ alkyl), -(C₁₋₆ alkylene)-CO-N(R¹¹)-O-R¹¹, -(C₁₋₆ alkylene)-O-CO-N(R¹¹)-R¹¹, -(C₁₋₆ alkylene)-N(R¹¹)-CO-N(R¹¹)-R¹¹, -(C₁₋₆ alkylene)-N(R¹¹)-C(=N-R¹¹)-N(R¹¹)-R¹¹, -(C₁₋₆ alkylene)-SO₃-R¹¹, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R³, wherein said alkyl, said alkenyl, said alkynyl,
   and any alkylene group comprised in any of the aforementioned R¹ groups are each optionally substituted with one or more -OH, and further wherein each R¹¹ is independently selected from hydrogen and C₁₋₆ alkyl;
   R² is selected from hydrogen, C₁₋₆ alkyl, C₂₋₆ alkenyl, and C₂₋₆ alkynyl; and
   each R³ is independently selected from C₁₋₆ alkyl, C₂₋₆ alkenyl, C₂₋₆ alkynyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -S(C₁₋₆ alkylene)-SH, -S(C₁₋₆ alkylene)-S(C₁₋₆ alkyl), -(C₁₋₆ alkylene)-SH, -(C₁₋₆ alkylene)-S(C₁₋₆ alkyl), -NH₂, -NH(C₁₋₆ alkyl), -N(C₁₋₆ alkyl)(C₁₋₆ alkyl), -NH-OH, -N(C₁₋₆ alkyl)-OH, -NH-O(C₁₋₆ alkyl), -N(C₁₋₆ alkyl)-O(C₁₋₆ alkyl), =NH, =N(C₁₋₆ alkyl), halogen, C₁₋₆ haloalkyl, -O-(C₁₋₆ haloalkyl), -CN, -NO₂, -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), -SO-(C₁₋₆ alkyl), -(C₀₋₄ alkylene)-carbocyclyl, and -(C₀₋₄ alkylene)-heterocyclyl, wherein the carbocyclyl group in said -(C₀₋₄ alkylene)-carbocyclyl and the heterocyclyl group in said -(C₀₋₄ alkylene)-heterocyclyl are each optionally substituted with one or more groups independently selected from C₁₋₆ alkyl, C₂₋₆ alkenyl, C₂₋₆ alkynyl, halogen, C₁₋₆ haloalkyl, -O-(C₁₋₆ haloalkyl), -CN, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), and -SO-(C₁₋₆ alkyl);
   or a pharmaceutically acceptable salt thereof,
   for use in therapy.
2. The compound for use according to item 1, wherein R¹ is selected from hydrogen, C₁₋₈ alkyl, C₂₋₈ alkenyl, C₂₋₈ alkynyl, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, -(C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R³, and further wherein said alkyl, said alkenyl, said alkynyl, and any alkylene group in any of the aforementioned groups are each optionally substituted with one or more -OH.
3. The compound for use according to item 1 or 2, wherein R¹ is selected from hydrogen, methyl, ethyl, n-propyl, isopropyl, n-butyl, isobutyl, sec-butyl, tert-butyl, n-pentyl, n-hexyl, -CH₂-CH=CH-CH₃, -CH₂-C≡CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), -CH(-OH)-(4-methoxyphenyl), - CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), -CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), - CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, -CH₂-(2-nitrocycloprop-1-yl), -CH₂-OH, -CH₂CH₂-OH, -CH₂-O-CH₃, -CH₂CH₂-O-CH₃, -CH₂CH₂-O-CH₂CH₃, -CH(-CH₃)-OH, -C(-CH₃)(-OH)-CH₃, -CH(-OH)-CH(-CH₃)-CH₃, -CH(-OH)-CH(-CH₃)-CH₂-CH=CH-CH₃, -CH(-OH)-C≡CH, -CH₂-SH, -CH₂CH₂-SH, -CH₂CH₂-S-CH₃, -CH₂CH₂-S-CH₂CH₃, -CH₂-SO₃H, -CH₂-COOH, -CH₂CH₂-COOH, -CH(-OH)-COOH, -CH(-OH)-CH₂-COOH, -CH(-CH₃)-COOH, -CH(-CH₃)-CH₂-COOH, -CH₂-CO-NH₂, -CH₂CH₂-CO-NH₂, -CH(-OH)-CO-NH₂, -CH₂-NH-CO-NH₂, -(CH₂)₂-NH-CO-NH₂, -(CH₂)₃-NH-CO-NH₂, -(CH₂)₂-CO-NH-OH, -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, -(CH₂)₂-C(-OH)-C(-CH₂-OH)-NH₂, -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, -CH₂-C(-OH)-CH₂CH₂-NH-C(=NH)-NH₂, and -CH(-OH)-CH(-OH)-CH₂-O-CO-NH₂.
4. The compound for use according to any one of items 1 to 3, wherein R² is hydrogen.
5. The compound for use according to any one of items 1 to 4, wherein said compound has the following absolute configuration: 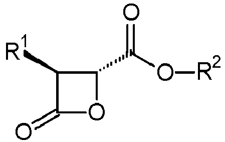
6. The compound for use according to item 1, wherein said compound is any one of the following compounds or a pharmaceutically acceptable salt thereof: 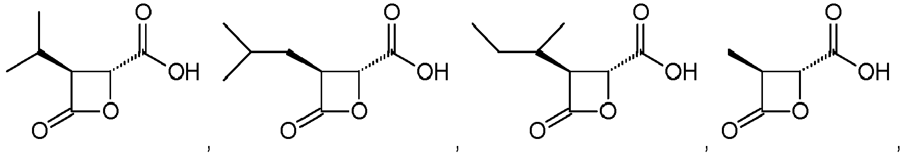 or 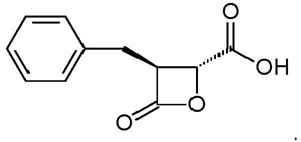
7. The compound for use according to item 1, wherein said compound is 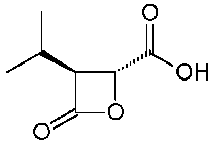 or a pharmaceutically acceptable salt thereof.
8. A pharmaceutical composition comprising a compound as defined in any one of items 1 to 7 and a pharmaceutically acceptable excipient.
9. A compound as defined in any one of items 1 to 7 or the pharmaceutical composition of item 8 for use in the treatment or prevention of cancer, an infectious disease, an inflammatory disease, or an autoimmune disease.
10. The compound for use according to item 9 or the pharmaceutical composition for use according to item 9, for use in the treatment or prevention of cancer, wherein said cancer is selected from lung cancer, renal cancer, gastro-intestinal cancer, stomach cancer, colorectal cancer, colon cancer, malignant familial adenomatous polyposis, anal cancer, genitourinary cancer, bladder cancer, liver cancer, pancreatic cancer, ovarian cancer, cervical cancer, endometrial cancer, vaginal cancer, vulvar cancer, prostate cancer, testicular cancer, biliary tract cancer, hepatobiliary cancer, neuroblastoma, brain cancer, breast cancer, head and/or neck cancer, skin cancer, melanoma, Merkel-cell carcinoma, epidermoid cancer, squamous cell carcinoma, bone cancer, fibrosarcoma, Ewing's sarcoma, malignant mesothelioma, esophageal cancer, laryngeal cancer, mouth cancer, thymoma, neuroendocrine cancer, hematological cancer, leukemia, lymphoma, and multiple myeloma.
11. The compound for use according to item 10 or the pharmaceutical composition for use according to item 10, wherein said cancer is multiple myeloma.
12. The compound for use according to item 9 or the pharmaceutical composition for use according to item 9, for use in the treatment or prevention of an infectious disease, wherein said infectious disease is selected from a bacterial infectious disease, a protozoan infectious disease, a fungal infectious disease, and a helminth infectious disease.
13. The compound for use according to item 12 or the pharmaceutical composition for use according to item 12, for use in the treatment or prevention of a bacterial infectious disease, wherein said bacterial infectious disease is caused by Gram-positive bacteria;
   preferably wherein said Gram-positive bacteria are selected from: *Listeria* spp., including e.g. *Listeria monocytogenes* or *Listeria welshimeri*; *Staphylococcus* spp., including e.g. *Staphylococcus aureus*, *Staphylococcus epidermidis, Staphylococcus saprophyticus, Staphylococcus lugdunensis, Staphylococcus schleiferi*, or *Staphylococcus caprae*; *Streptococcus* spp., including e.g. *Streptococcus pneumoniae, Streptococcus viridans*, *Streptococcus pyogenes*, or *Streptococcus agalactiae*; *Enterococcus* spp., including e.g. *Enterococcus faecalis* or *Enterococcus faecium*; *Bacillus* spp., including e.g. *Bacillus licheniformis, Bacillus subtilis*, *Bacillus anthracis*, *Bacillus cereus*, *Bacillus thuringiensis*, or *Bacillus larvae*; *Mycobacterium* spp., including e.g. *Mycobacterium tuberculosis*, *Mycobacterium bovis*, *Mycobacterium leprae*, *Mycobacterium ulcerans, Mycobacterium kanasasii, Mycobacterium avium, Mycobacterium paratuberculosis, Mycobacterium scrofulaceam, Mycobacterium microti, Mycobacterium africanum, Mycobacterium canettii, Mycobacterium intracellulare, Mycobacterium simiae, Mycobacterium szulgai, Mycobacterium xenopi*, *Mycobacterium fortuitum*, *Mycobacterium chelonei*, or *Mycobacterium marinum*; *Nocardia* spp., including e.g. *Nocardia asteroids;* and *Rhodococcus* spp., including e.g. *Rhodococcus equi.*
14. The compound for use according to item 12 or the pharmaceutical composition for use according to item 12, for use in the treatment or prevention of a bacterial infectious disease, wherein said bacterial infectious disease is caused by Gram-negative bacteria;
   preferably wherein said Gram-negative bacteria are selected from: *Neisseria* spp., including e.g. *Neisseria gonorrhoeae* or *Neisseria meningitides*; *Moraxella* spp., including e.g. *Moraxella catarrhalis*; *Hemophilus* spp., including e.g. *Hemophilus influenzae*; *Klebsiella* spp., including e.g. *Klebsiella pneumoniae*; *Legionella* spp., including e.g. *Legionella pneumophila*; *Pseudomonas* spp., including e.g. *Pseudomonas aeruginosa* or *Pseudomonas putida*; *Escherichia* spp., including e.g. *Escherichia coli*; *Proteus* spp., including e.g. *Proteus mirabilis*; *Enterobacter* spp., including e.g. *Enterobacter cloaceae*; *Serratia* spp., including e.g. *Serratia marcescens*; *Helicobacter* spp., including e.g. *Helicobacter pylori*; and *Salmonella* spp., including e.g. *Salmonella enteritidis* or *Salmonella typhi.*
15. The compound for use according to item 12 or the pharmaceutical composition for use according to item 12, for use in the treatment or prevention of a protozoan infectious disease, wherein said protozoan infectious disease is caused by a protozoan of the phylum Apicomplexa;
   preferably wherein said protozoan infectious disease is caused by a protozoan selected from: *Plasmodium* spp., including e.g. *Plasmodium falciparum*, *Plasmodium malariae*, *Plasmodium vivax*, *Plasmodium ovale*, or *Plasmodium knowlesi*; *Leishmania* spp., including e.g. *Leishmania major, Leishmania tropica, Leishmania aethiopica*, *Leishmania mexicana*, *Leishmania braziliensis*, *Leishmania donovani*, *Leishmania infantum,* or *Leishmania chagasi*; *Trypanosoma* spp., including e.g. *Trypanosoma brucei*, *Trypanosoma cruzi, Trypanosoma simiae, Trypanosoma avium, Trypanosoma congolense, Trypanosoma equinum, Trypanosoma equiperdum*, *Trypanosoma evansi*, or *Trypanosoma suis*; *Babesia* spp., including e.g. *Babesia microti* or *Babesia bigemina*; and *Toxoplasma* spp., including e.g. *Toxoplasma gondii.*
16. The compound for use according to item 12 or the pharmaceutical composition for use according to item 12, for use in the treatment or prevention of a fungal infectious disease, wherein said fungal infectious disease is caused by a fungus selected from: *Aspergillus* spp., including e.g. *Aspergillus fumigatus*, *Aspergillus flavus*, or *Aspergillus clavatus*; *Candida* spp., including e.g. *Candida albicans*, *Candida stellatoidea*, *Candida tropicalis*, *Candida pseudotropicalis*, *Candida krusei, Candida parapsilosis,* or *Candida guilliermondii*; *Cryptococcus* spp., including e.g. *Cryptococcus neoformans*, *Cryptococcus laurentii*, *Cryptococcus albidus*, or *Cryptococcus gattii*; *Histoplasma* spp., including e.g. *Histoplasma capsulatum*; *Pneumocystis* spp., including e.g. *Pneumocystis jirovecii* or *Pneumocystis carinii*; *Stachybotrys* spp., including e.g. *Stachybotrys chartarum*; *Basidiobolus* spp., including e.g. *Basidiobolus ranarum*; *Blastomyces* spp., including e.g. *Blastomyces dermatitidis*; *Coccidioides* spp., including e.g. *Coccidioides immitis* or *Coccidioides posadasii*; *Conidiobolus* spp., including e.g. *Conidiobolus coronatus* or *Conidiobolus incongruous*; and *Madurella* spp., including e.g. *Madurella mycetomatis* or *Madurella grisea.*
17. The compound for use according to item 12 or the pharmaceutical composition for use according to item 12, for use in the treatment or prevention of a helminth infectious disease, wherein said helminth infectious disease is caused by a roundworm, a tapeworm, a trematode, or an acanthocephalan; preferably wherein said helminth infectious disease is caused by a helminth selected from: *Wuchereria* spp., including e.g. *Wuchereria bancrofti*; *Brugia* spp., including e.g. *Brugia malayi*; *Onchocerca* spp., including e.g. *Onchocerca volvulus*; *Ascaris* spp., including e.g. *Ascaris lumbricoides*; *Trichuris* spp., including e.g. *Trichuris trichiura*, *Trichuris campanula*, *Trichuris serrata*, *Trichuris suis*, *Trichuris muris*, or *Trichuris vulpis*; *Necatorspp.,* including e.g. *Necator americanus*; *Ancylostoma* spp., including e.g. *Ancylostoma duodenale*; *Trichostrongylus* spp., including e.g. *Trichostrongylus affinis*, *Trichostrongylus sigmodontis*, *Trichostrongylus tenuis,* or *Trichostrongylus retortaeformis*; *Dracunculus* spp., including e.g. *Dracunculus medinensis*; *Baylisascaris* spp., including e.g. *Baylisascaris procyonis, Baylisascaris melis*, *Baylisascaris transfuga*, *Baylisascaris columnaris, Baylisascaris devosi, Baylisascaris laevis, Baylisascaris shroederi,* or *Baylisascaris potosis*; *Echinococcus* spp., including e.g. *Echinococcus granulosus, Echinococcus multilocularis, Echinococcus vogeli,* or *Echinococcus oligarthrus; Hymenolepis* spp., including e.g. *Hymenolepis diminuta, Hymenolepis nana, Hymenolepis apodemi, Hymenolepis asymetrica, Hymenolepis horrida, Hymenolepis rymzhanovi,* or *Hymenolepis microstoma; Taenia* spp., including e.g. *Taenia multiceps, Taenia glomerata, Taenia brauni, Taenia asiatica, Taenia bubesei, Taenia crassiceps, Taenia hydatigena, Taenia mustelae, Taenia ovis, Taenia pisiformis, Taenia rileyi, Taenia saginata, Taenia solium, Taenia taeniaeformis,* or *Taenia serialis; Gastrodiscoides* spp., including e.g. *Gastrodiscoides hominis* or *Gastrodiscoides discoides; Watsonius* spp., including e.g. *Watsonius watsoni*; *Clonorchis* spp., including e.g. *Clonorchis sinensis*; *Fasciola* spp., including e.g. *Fasciola hepatica* or *Fasciola gigantica*; *Fasciolopsis* spp., including e.g. *Fasciolopsis buski; Opisthorchis* spp., including e.g. *Opisthorchis chabaudi, Opisthorchis felineus, Opisthorchis gomtii, Opisthorchis parasiluri,* or *Opisthorchis viverrini; Paragonimus* spp., including e.g. *Paragonimus westermani*, *Paragonimus africanus, Paragonimus caliensis, Paragonimus compactus, Paragonimus ecuadoriensis, Paragonimus heterotremus, Paragonimus hueitugensis, Paragonimus iloktsuenensis*, *Paragonimus kellicotti, Paragonimus mexicanus, Paragonimus miyazakii, Paragonimus ohirai, Paragonimus pulmonalis, Paragonimus peruvianus, Paragonimus sadoensis, Paragonimus skrjabini*, or *Paragonimus uterobilateralis; Schistosoma* spp., including e.g. *Schistosoma bomfordi, Schistosoma bovis, Schistosoma curassoni, Schistosoma datta, Schistosoma edwardiense, Schistosoma guineensis, Schistosoma haematobium, Schistosoma harinasutai, Schistosoma hippopotami, Schistosoma incognitum, Schistosoma indicum, Schistosoma intercalatum, Schistosoma japonicum, Schistosoma kisumuensis, Schistosoma leiperi, Schistosoma malayensis, Schistosoma mansoni, Schistosoma margrebowiei, Schistosoma mattheei, Schistosoma mekongi, Schistosoma ovuncatum, Schistosoma nasale*, *Schistosoma rodhaini, Schistosoma sinensium, Schistosoma spindale,* or *Schistosoma turkestanicum*; *Moniliformis* spp., including e.g. *Moniliformis moniliformis; Toxocara* spp., including e.g. *Toxocara canis* or *Toxocara cati*; *Enterobius* spp., including e.g. *Enterobius vermicularis* or *Enterobius gregorii;* and *Strongyloides* spp., including e.g. *Strongyloides stercoralis, Strongyloides fuelleborni,* or *Strongyloides kellyi.*
18. The compound for use according to item 9 or the pharmaceutical composition for use according to item 9, for use in the treatment or prevention of an inflammatory disease or an autoimmune disease, wherein said inflammatory or autoimmune disease is selected from ischemia, autoimmune bleeding disorder, septic shock, rheumatoid arthritis, inflammatory bowel disease, systemic lupus erythematosus (SLE), multiple sclerosis, asthma, osteoarthritis, osteoporosis, a fibrotic disease, dermatosis, psoriasis, atopic dermatitis, ultraviolet radiation (UV)-induced skin damage, psoriatic arthritis, ankylosing spondylitis, tissue and organ rejection, Alzheimer's disease, stroke, atherosclerosis, restenosis, diabetes, glomerulonephritis, Hodgkin's disease, cachexia, inflammation associated with infection, inflammation associated with viral infection, acquired immune deficiency syndrome (AIDS), adult respiratory distress syndrome, and ataxia telangiectasia.
19. A plant protection composition comprising a compound of formula (I) as defined in any one of items 1 to 7 or an agriculturally acceptable salt thereof.
20. A method of controlling a plant disease, the method comprising applying a compound of formula (I) as defined in any one of items 1 to 7 or an agriculturally acceptable salt thereof or the plant protection composition of item 19.
21. The method of item 20, wherein the compound or the plant protection composition is applied by treating a plant or a part thereof with the compound or the composition, by treating the soil on which a plant is cultivated with the compound or the composition, by hydroponic treatment of a plant with the compound or the composition, or by treating a plant seed or bulb with the compound or the composition.
22. The method of item 20 or 21, wherein the plant disease is caused by a plant pathogenic organism selected from a bacterium, a protozoan, a fungus, an oomycete, a nematode, and an insect.
23. The method of any one of items 20 to 22, wherein the plant is a crop plant which is preferably selected from rice, wheat, corn/maize, sugarcane, potatoes, manioc, sorghum, millets, peanuts, beans, sweet potatoes, barleys, bananas, soybeans, tomatoes, grapes, apples, onions, cucumbers, garlic, melons, mangos, spinach, strawberries, peaches, nectarines, lettuce, chicory, peas, coffee, tea, tobacco, and cotton.
24. A disinfectant comprising a compound of formula (I) as defined in any one of items 1 to 7 or a salt thereof.
25. Non-therapeutic use of a compound of formula (I) as defined in any one of items 1 to 7 or a salt thereof, or the disinfectant of item 24, for disinfecting or sterilizing an inanimate object.
26. A method of disinfecting or sterilizing an inanimate object, the method comprising applying a compound of formula (I) as defined in any one of items 1 to 7 or a salt thereof, or the disinfectant of item 24, to said inanimate object.
27. The use of item 25 or the method of item 26, wherein the inanimate object is a technical device, preferably wherein the technical device is selected from a catheter, a medical implant, a surgical instrument, contact lenses, and a food-processing device.
28. *In vitro* use of a compound as defined in any one of items 1 to 7 as a proteasome inhibitor.

The invention is furthermore illustrated by the following examples which are not to be construed as limiting.

### EXAMPLES

The present inventors have isolated compound **1** ("IOC") as the product of ioc/leu biosynthetic gene cluster from the culture supernatants of *P. luminescens* subsp. *laumondii* TT01, *Xenorhabdus nematophila* ATCC 19061, and *Xenorhabdus szentirmaii* DSM 16338 wild-type strains (Example 1) and confirmed its identity by mass spectrometry and comparison with a synthetically prepared compound (Preparative Example 1 and Example 2). Compound **1** was shown by the present inventors to be a potent inhibitor of the 20S proteasome, in particular the eukaryotic 20S proteasome (Example 2).

### General experimental procedures

All chemicals were purchased from Sigma-Aldrich, Acros Organics, or Iris BIOTECH. Isotope-labeled chemicals were purchased from Cambridge Isotope Laboratories, Inc. Genomic DNA of selected *Xenorhabdus* and *Photorhabdus* strains were isolated using the Qiagen Gentra Puregene Yeast/Bact Kit. DNA polymerases (Taq, Phusion, and Q5) and restriction enzymes were purchased from New England Biolabs or Thermo Fisher Scientific. DNA primers were purchased from Eurofins MWG Operon. PCR amplifications were carried out on thermocyclers (SensoQuest). Polymerases were used according to the manufacturers' instructions. DNA purification was performed from 1% TAE agarose gel using Invisorb^{®} Spin DNA Extraction Kit (STRATEC Biomedical AG). Plasmids in *E. coli were* isolated by alkaline lysis. HPLC-UV-MS analysis was conducted on an UltiMate 3000 system (Thermo Fisher) coupled to an AmaZonX mass spectrometer (Bruker) with an ACQUITY UPLC BEH C18 column (130 Å, 2.1 mm x 100 mm, 1.7 µm particle size, Waters) at a flow of 0.6 mL/min (5-95% acetonitrile/water with 0.1% formic acid, v/v, 16 min, UV detection wavelength 190-800 nm). HPLC-UV-HRMS analysis was conducted on an UltiMate 3000 system (Thermo Fisher) coupled to an Impact II qTof mass spectrometer (Bruker) with an ACQUITY UPLC BEH C18 column (130 Å, 2.1 mm × 100 mm, 1.7 µm particle size, Waters) at a flow of 0.4 mL/min (5-95% acetonitrile/water with 0.1% formic acid, v/v, 16 min, UV detection wavelength 190-800 nm). TLC was performed using aluminum plates precoated with silica gel 60 F₂₅₄ and visualized by UV light, KMnO4, phosphomolybdic acid, sulfuric acid, anisaldehyde or Cerium Ammonium Molybdate stains, followed by heating. Silica flash purification was performed on a Biotage SP1^{™} flash purification system (Biotage, Uppsala, Sweden) by a silica gel (SiO₂) column coupling with a UV detector. Freeze drying was performed by BUCHI Lyovapor^{™} L-300 Continuous. NMR experiments were acquired on a Bruker AVANCE 500 MHz spectrometer equipped with a 5 mm cryoprobe.

### Preparative Example 1: Synthesis of compound 1.

### 2R,3S-IOC (compound 1) was synthesized as follows:

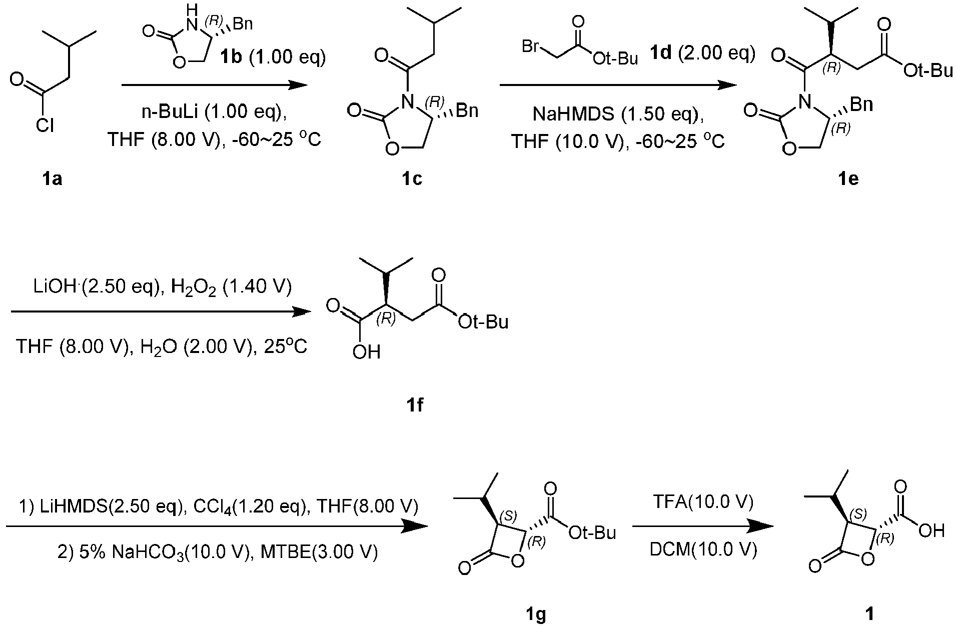

### Synthetic procedure for compound 1c

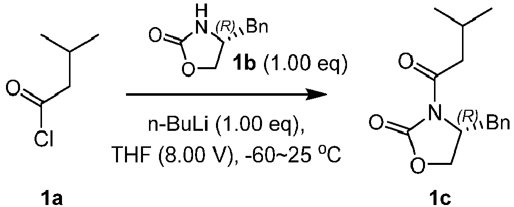

### Reagents:

| **Reagent/reactant/solvent** | **Molecular weight** | **Density at 25°C (g/mL)** | **Amount** | **mMol (mol)** | **Equivalent** |
|---|---|---|---|---|---|
| Compound 1a | 120 | / | 2.02 g | 16.7 | 1.10 |
| Compound 1b | 177 | / | 2.70 g | 15.2 | 1.00 |
| n-BuLi | 64.1 | / | 0.98 g | 15.2 | 1.00 |
| THF | / | 0.89 | 21.6 mL | / | / |

### Procedure:

1. Set up a reactor R-1.
2. Charge compound 1b (2.70 g, 15.2 mmol, 1.00 eq) and THF (21.6 mL) into R-1 at 20 ~ 25 °C.
3. Charge n-BuLi (2.50 M, 6.09 mL, 1.00 eq) into R-1 at -55 ~ -65 °C.
4. Stir the mixture at -55 ~ -65 °C for 0.5 h.
5. Charge the compound 1a (2.02 g, 16.7 mmol, 2.06 mL, 1.10 eq) into R-1 at -55 ~ -65 °C.
6. Stir the mixture at 20 ~ 25 °C for 16 h.
7. TLC (petroleum ether/ethyl acetate = 3/1, R_{f} =0.43) shows the reaction is completed.
8. MeOH (5.00 mL) was added to the reaction mixture and the mixture was concentrated under reduced pressure.
9. The residue was dissolved in ethyl acetate (5.00 mL), washed with water (5.00 mL), 1N hydrochloric acid (5.00 mL) and saturated brine (5.00 mL), and dried over sodium sulfate.
10. The residue was purified by column chromatography (SiO₂, petroleum ether/ethyl acetate=50/1 to 20/1).
11. The mixture was cooled down to room temperature and concentrated in vacuum to afford compound 1c (yellow oil, 3.50 g, 12.1 mmol, 79.1% yield, 90% purity).

### Synthetic procedure for compound 1e

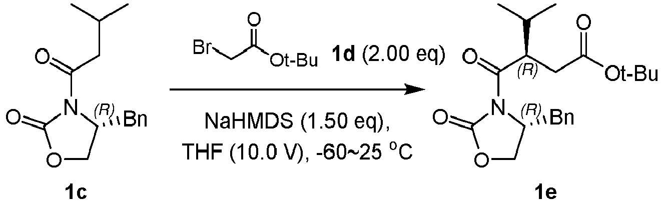

### Reagents:

| **Reagent/reactant/solvent** | **Molecular weight** | **Density at 25 °C (g/mL)** | **Amount** | **mMol (mol)** | **Equivalent** |
|---|---|---|---|---|---|
| Compound 1c | 261 | / | 3.50 g | 13.4 | 1.00 |
| Compound 1d | 195 | / | 5.23 g | 26.8 | 2.00 |
| NaHMDS | 183 | / | 3.68 g | 20.1 | 1.50 |
| THF | / | 0.89 | 35.0 mL | / | / |

### Procedure:

1. Set up a reactor R-1.
2. Charge compound 1c (3.50 g, 13.4 mmol, 1.00 *eq)* and THF (35.0 mL) into R-1 at 20 ~ 25 °C.
3. Charge NaHMDS (1.00 M, 20.1 mL, 1.50 *eq)* into R-1 at -60 ~ -65 °C.
4. Stir the mixture at -60 ~ -65 °C for 0.5 h.
5. Charge compound 1d (5.23 g, 26.8 mmol, 3.96 mL, 2.00 *eq)* into R-1 at -60 ~ -65 °C.
6. Stir the mixture at 20 ~ 25 °C for 16 h.
7. TLC (petroleum ether/ethyl acetate = 2/1, R_{f} =0.40) shows the reaction is completed.
8. The reaction was quenched by saturated aqueous ammonium chloride solution (7.00 ml) and extracted with EtOAc (7.00 mL*2).
9. The mixture was concentrated in vacuum to afford compound 1e (off-white solid, 3.00 g, crude).

¹H NMR: compound 1e, 400 MHz CDCl₃
δ_{H}=7.33-7.25 (m, 5H) 4.66-4.61 (m, 1H) 4.15-4.12 (m, 3H) 3.35-3.31 (m, 1H) 2.84-2.69 (m, 2H) 2.45-2.40 (m, 1H) 1.98-1.95(m, 1H) 1.40 (s, 9H) 1.00-0.88 (m, 6H)

### Synthetic procedure for compound 1f

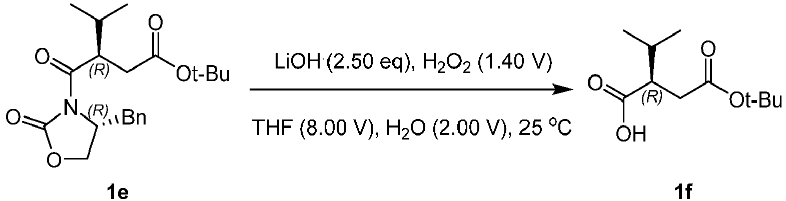

### Reagents:

| **Reagent/reactant/solvent** | **Molecular weight** | **Density at 25 °C** (g/mL) | **Amount** | **mMol (mol)** | **Equivalent** |
|---|---|---|---|---|---|
| Compound 1e | 375 | / | 3.00 g | 7.99 | 1.00 |
| LiOH.H₂O | 41.9 | / | 0.84 g | 20.0 | 2.50 |
| H₂O₂ | 34.0 | / | 4.24 mL | 44.1 | / |
| THF | / | 0.89 | 24.0 mL | / | / |
| H₂O | / | 1.00 | 6.00 mL | / | / |

### Procedure:

1. Set up a reactor R-1.
2. Charge THF (24.0 mL) and H₂O (6.00 mL) into R-1 at 0 ~ 5 °C.
3. Charge H₂O₂ (5.00 g, 44.1 mmol, 4.24 mL, 30.0% purity, 5.52 *eq)* into R-1 at 0 ~ 5 °C.
4. Charge compound 1e (3.00 g, 7.99 mmol, 1.00 *eq)* into R-1 at 0 ~ 5°C.
5. Stir the mixture at 0 ~ 5 °C for 0.5 h.
6. Charge LiOH.H₂O (2.00 M, 9.99 mL, 2.50 *eq)* into R-1 at 0 ~ 5 °C.
7. Stir the mixture at 20 ~ 25 °C for 12 h.
8.TLC (petroleum ether/ethyl acetate = 3/1, R_{f} =0.20) shows the reaction is completed.
9. The reaction mixture was cooled to 0 °C and quenched with Na₂S₂O₃ (10.0 mL).
10. The mixture was cooled down to room temperature. The residue was diluted with water (10.0 mL), washed with MTBE (10.0 mL*2), filtered, and concentrated in vacuum.
11. The residue was purified by column chromatography (SiO₂, petroleum ether/ethyl acetate=50/1 to 20/1).
12. The mixture was concentrated in vacuum to afford compound 1f (yellow oil, 1.70 g, 8.13 mmol, 78.9% yield).

¹H NMR: compound 1f, 400 MHz CDCl₃
δ_{H}=2.68-2.56 (m, 2H) 2.35-2.30 (m, 1H) 2.07-1.99 (m, 1H) 1.41(s, 9H) 0.96-0.92 (m, 6H)

### Synthetic procedure for compound 1g

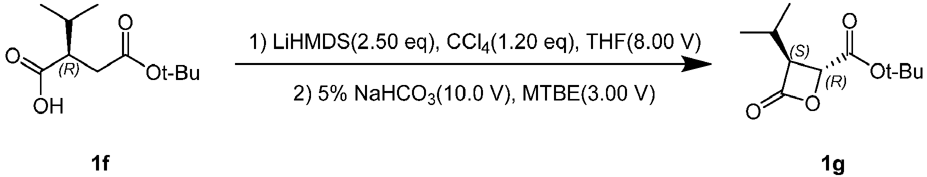

### Reagents:

| **Reagent/reactant/solvent** | **Molecular weight** | **Density at 25 °C (g/mL)** | **Amount** | **mMol (mol)** | **Equivalent** |
|---|---|---|---|---|---|
| Compound 1f | 216 | / | 1.10 g | 5.09 | 1.00 |
| LiHMDS | 167 | / | 2.13 g | 12.7 | 2.50 |
| CCl₄ | 153 | / | 0.94 g | 6.10 | 1.20 |
| THF | / | 0.89 | 8.80 mL | / | / |

### Procedure:

1. Set up a reactor R-1.
2. Charge compound 1f (1.10 g, 5.09 mmol, 1.00 *eq)* and THF (8.80 mL) into R-1 at 0 ~ 5 °C.
3. Charge LiHMDS (1.00 M, 12.7 mL, 2.50 *eq)* into R-1 at -60 ~ -65 °C.
4. Stir the mixture at -60 ~ -65 °C for 1 h.
5. Charge CCl₄ (938 mg, 6.10 mmol, 586 uL, 1.20 *eq)* into R-1 at -60 ~ -65 °C.
6. Stir the mixture at -60 ~ -65 °C for 2 h.
7. TLC (petroleum ether/ethyl acetate = 3/1, R_{f} =0.50) shows the reaction is completed.
8. The mixture was cooled down to room temperature and concentrated under reduced pressure. The residue was diluted with water (5.00mL), washed with MTBE (5.00mL*2), and acidified with NaHCO₃ (5.00mL). The resulting precipitate was filtered off, dried-up on air overnight, and then dissolved in MTBE (5.00mL).
9. The residue was purified by column chromatography (SiO₂, petroleum ether/ethyl acetate=50/1 to 20/1).
10. The mixture was concentrated in vacuum to afford compound 1g (yellow solid, 0.20 g, crude).

### Synthetic procedure for compound 1

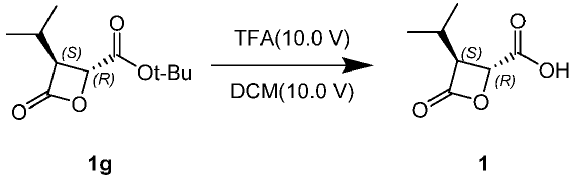

### Reagents:

| **Reagent/reactant/solvent** | **Molecular weight** | **Density at 25 °C (g/mL)** | **Amount** | **mMol (mol)** | **Equivalent** |
|---|---|---|---|---|---|
| Compound 1g | 214 | / | 0.50 g | 2.33 | 1.00 |
| TFA | 114 | / | 5.00 mL | / | / |
| DCM | / | 1.32 | 5.00 mL | / | / |

### Procedure:

1. Set up a reactor R-1.
2. Charge compound 1g (0.50 g, 876 umol, 1.00 *eq)* and DCM (5.00 mL) into R-1 at 0 ~ 5 °C.
3. Charge TFA (5.00 mL) into R-1 at 0 ~ 5 °C.
4. Stir the mixture at 0 ~ 5 °C for 12 h.
5. TLC (petroleum ether/ethyl acetate = 1/1, R_{f} =0.1) shows the reaction is completed.
6. The mixture was cooled to 0 ~ 5 °C and concentrated in vacuum.
8. The residue was purified by column chromatography (SiO₂, petroleum ether/ethyl acetate=50/1 to 2/1).
9. The mixture was cooled to 0 ~ 5°C and concentrated in vacuum to afford compound 1 (brown oil, 30.0 mg, 1.89 mmol).

¹H NMR: compound 1, 400 MHz DMSO
δ_{H}=4.70-4.69 (d, 1H) 3.75-3.65 (dd, 1H) 2.30-2.16 (m, 1H) 1.16-1.05 (m, 6H)
HR-MS-ESI⁻: compound 1
Detected mass 157.0508 [M - H]-, calculated mass 157.0506 [M - H]-, error (ppm) -1.1, C₇H₉O₄ [M - H]-

### Example 1: Identification of compound 1 as the product of ioc/leu BCG

*P. luminescens* subsp. *laumondii* TT01, *Xenorhabdus nematophila* ATCC 19061, and *Xenorhabdus szentirmaii* DSM 16338 wild-type strains were cultured in various media. By HPLC-HRMS analysis of the culture supernatant from Sf-900 (a serum-free insect cell medium) with a negative ion mode, a peak with an *m*/*z* 157.0508 [M - H]- was detected, whose deduced sum formula, C₇H₉O₄, coincides with that of compound **1**. Finally, (2*R*,3*S*)-**1** was synthesized and showed identical retention time and MS/MS fragmentation patterns with compound **1** in HPLC-HRMS **(****Figure 3**), which confirmed the planar structure and stereochemistry of compound **1**.

### Example 2: Inhibition of 20S-proteasome by the compounds of formula (I)

Compound 1 inhibits the yeast 20S proteasome with an IC₅₀ value of 6.2 µM for the β5 subunit (**Fig. 1b**), whereas it has low binding affinities for β1 (625 µM) and β2 (60 µM), as determined according to the assay described hereinbelow. The crystal structure of compound **1** in complex with the yeast 20S proteasome at 3.0 Å (PDB ID 7O2L) was solved. The electron density map displayed compound **1** covalently bound to Thr1Oγ of all active sites due to the high ligand concentrations used for crystal soaking (**Fig. 1c**). However, since compound **1** lacks strong interactions with protein residues in the caspase- and trypsin-like binding channels, the 2*F*ₒ-*F*_{c}-map for the ligand is diffuse at β1 and β2. In contrast, compound **1** is well defined in the β5 subunit and adopts a similar conformation as observed for homobelactosin C (**Fig. 1d**). Hereby, the acyl-oxygen atom of compound **1** derived from β-lactone ring-opening is stabilized by the oxyanion hole (Gly47NH), whereas the generated hydroxyl group is hydrogen-bonded to the carbonyl oxygen of residue 19. In similarity to NH6 in belactosin products, the carboxylate group of compound **1** interacts with the threonine N-terminus and displaces the nucleophilic water molecule (**Fig. 1c**), thereby preventing hydrolysis of the acyl enzyme complex and explaining its inhibitory effect. Furthermore, the isopropyl moiety of compound **1** at the P1 site is stabilized by Ala20, Met45, and Ala49 in the chymotrypsin-like channel. Although these interactions are present in other β-lactone containing compounds, they adopt a diverse and unpredictable mode of binding. Without nitrogen atoms and extension units, compound **1** is considered to feature the minimal scaffold for proteasome inhibition.

### IC₅₀ value determination with the purified yeast 20S proteasome core particle (yCP)

The concentration of purified yCP was determined spectrophotometrically at 280 nm. yCP (final concentration: 0.05 mg/mL in 100 mM Tris-HCI, pH 7.5) was mixed with DMSO as a control or serial dilutions of the compound **1** in DMSO, thereby not surpassing a final concentration of 10% (v/v) DMSO. After an incubation time of 45 min at room temperature, fluorogenic substrates Boc-Leu-Arg-Arg-AMC, Z-Leu-Leu-Glu-AMC, and Suc-Leu-Leu-Val-Tyr-AMC (final concentration of 200 µM) were added to measure the residual activity of caspase-like (C-L, β1 subunit), trypsin-like (T-L, β2 subunit), and chymotrypsin-like (ChT-L, β5 subunit), respectively. The assay mixture was incubated for another 60 min at RT and afterward diluted 1:10 in 20 mM Tris-HCI, pH 7.5. The AMC-molecules released by hydrolysis were measured in triplicate with a Varian Cary Eclipse Fluorescence Spectrophotometer (Agilent Technologies) at λ_{exc} = 360 nm and λₑₘ = 460 nm. Relative fluorescence units were normalized to the DMSO treated control. The calculated residual activities were plotted against the logarithm of the applied inhibitor concentration and fitted with GraphPad Prism 9. Half maximum inhibitory concentration (IC₅₀) values were deduced from the fitted data. They depend on enzyme concentration and are comparable within the same experimental settings. The corresponding data is shown in **Fig. 1b****.**

### Crystallization and structure determination of the yCP in complex with compound 1

Crystals of the yCP were grown in hanging drops at 20 °C as previously described (Groll, M. & Huber, R. in Methods Enzymol. Vol. 398 329-336 (Academic Press, 2005); Gallastegui, N. & Groll, M. Analysing properties of proteasome inhibitors using kinetic and X-ray crystallographic studies. Methods Mol. Biol. 832, 373-390 (2012)).The protein concentration used for crystallization was 40 mg/mL in Tris / HCI (20 mM, pH 7.5) and EDTA (1 mM). The drops contained 1 µL of protein and 1 µL of the reservoir solution [30 mM magnesium acetate, 100 mM 2-(N-morpholino)ethanesulfonic acid (pH 6.7) and 10% (wt/vol) 2-methyl-2,4-pentanediol]. Crystals appeared after two days and were incubated with compound **1** at final concentrations of 10 mM for at least 24 h. Droplets were then complemented with a cryoprotecting buffer [30% (wt/vol) 2-methyl-2,4-pentanediol, 15 mM magnesium acetate, 100 mM 2-(N-morpholino)ethanesulfonic acid, pH 6.9] and vitrified in liquid nitrogen. The dataset from the yCP:IOC complex was collected using synchrotron radiation (λ =1.0 Å) at the X06SA-beamline (Swiss Light Source, Villingen, Switzerland). X-ray intensities and data reduction were evaluated using the XDS program package (as in Table 2 shown below) (Kabsch, W. XDS. Acta Crystallogr. D Biol. Crystallogr. 66, 125-132 (2010)). Conventional crystallographic rigid body, positional, and temperature factor refinements were carried out with REFMAC5 (Schneider, T. R. & Sheldrick, G. M. Substructure solution with SHELXD. Acta Crystallogr. D Biol. Crystallogr. 58, 1772-1779 (2002)) using coordinates of the yCP structure as starting model (PDB ID 5CZ4, Huber, E. M. et al. A unified mechanism for proteolysis and autocatalytic activation in the 20S proteasome. Nat. Commun. 7, 10900 (2016)). For model building, the programs SYBYL and COOT (Emsley, P., Lohkamp, B., Scott, W. G. & Cowtan, K. Features and development of Coot. Acta Crystallogr. D Biol. Crystallogr. 66, 486-501 (2010)) were used. The final coordinates yielded excellent R factors, as well as geometric bond and angle values. Coordinates were confirmed to fulfill the Ramachandran plot.

**Table 2. Crystallographic data collection and refinement statistics.**

| | ***yCP: compound** 1* |
|---|---|
| **Crystal parameters** | |
| Space group | P2₁ |
| Cell constants | a = 135.1 Å |
| | b = 301.5 Å |
| | c= 144.3 Å |
| | β = 112.9 ° |
| CPs / AU^{a} | 1 |

| **Data collection** | |
|---|---|
| Beam line | X06SA, SLS |
| Wavelength (Å) | 1.0 |
| Resolution range (A)^{b} | 30-3.0 (3-1-3.0) |
| No. observations | 635463 |
| No. unique reflections^{c} | 203696 |
| Completeness (%)^{b} | 96.2 (98.6) |
| R_{merge} (%)^{b, d} | 8.5 (57.7) |
| I/ σ (I)^{b} | 9.1 (2.6) |

| **Refinement (REFMAC5)** | |
|---|---|
| Resolution range (Å) | 30-3.0 |
| No. refl. working set | 193349 |
| No. refl. test set | 10176 |
| No. non hydrogen | 49509 |
| No. of ligand atoms | 66 |
| Solvent (H₂O, ions, MES) | 138 |
| Rwork/R_{free} (%)^{e} | 17.8/21.5 |
| r.m.s.d. bond (Å) / angle (°)^{f} | 0.002 / 1.2 |
| Average B-factor (Å²) | 90.5 |
| Ramachandran Plot (%)^{g} | 97.4 / 2.3 / 0.3 |
| PDB accession code | 702L |

| | |
|---|---|
| ^{[a]} Asymmetric unit ^{[b]} The values in parentheses for resolution range, completeness, R_{merge} and l/σ (I) correspond to the highest resolution shell ^{[c]} Data reduction was carried out from a single crystal. Friedel pairs were treated as identical reflections ^{[d]} R_{merge}(I) = ΣₕₖₗΣⱼ \| I(hkl)ⱼ - <I(hkl)> \| / Σₕₖₗ Σⱼ I(hkl)ⱼ, where I(hkl)ⱼ is the j^{th} measurement of the intensity of reflection hkl and <I(hkl)> is the average intensity ^{[e]}R = Σₕₖₗ \| \|F_{obs}\| - \|F_{calc}\| \|/Σₕₖₗ \|F_{obs}\|, where Rfree is calculated without a sigma cut off for a randomly chosen 5% of reflections, which were not used for structure refinement, and R_{work} is calculated for the remaining reflections ^{[f]} Deviations from ideal bond lengths/angles ^{[g]} Percentage of residues in favored / allowed / outlier region | |

### Conclusions

These results demonstrate that the compounds of formula (I) provided herein, including compound 1, are potent inhibitors of the 20S proteasome. The compounds of formula (I) can thus be used, e.g. for the treatment or prevention of cancer or an infectious disease.

## Claims

1. A compound of formula (I) 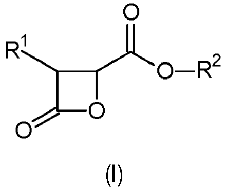 wherein:
R¹ is selected from hydrogen, C₁₋₈ alkyl, C₂₋₈ alkenyl, C₂₋₈ alkynyl, -(C₁₋₆ alkylene)-O-R¹¹, -(C₁₋₆ alkylene)-S-R¹¹, -(C₁₋₆ alkylene)-N(R¹¹)-R¹¹, -(C₁₋₆ alkylene)-CO-R¹¹, -(C₁₋₆ alkylene)-COO-R¹¹, -(C₁₋₆ alkylene)-O-CO-(C₁₋₆ alkyl), -(C₁₋₆ alkylene)-CO-N(R¹¹)-R¹¹, -(C₁₋₆ alkylene)-N(R¹¹)-CO-(C₁₋₆ alkyl), -(C₁₋₆ alkylene)-CO-N(R¹¹)-O-R¹¹, -(C₁₋₆ alkylene)-O-CO-N(R¹¹)-R¹¹, -(C₁₋₆ alkylene)-N(R¹¹)-CO-N(R¹¹)-R¹¹, -(C₁₋₆ alkylene)-N(R¹¹)-C(=N-R¹¹)-N(R¹¹)-R¹¹, -(C₁₋₆ alkylene)-SO₃-R¹¹, -(C₀₋₆ alkylene)-carbocyclyl, and -(C₀₋₆ alkylene)-heterocyclyl, wherein the carbocyclyl group in said -(C₀₋₆ alkylene)-carbocyclyl and the heterocyclyl group in said -(C₀₋₆ alkylene)-heterocyclyl are each optionally substituted with one or more groups R³, wherein said alkyl, said alkenyl, said alkynyl, and any alkylene group comprised in any of the aforementioned R¹ groups are each optionally substituted with one or more -OH, and further wherein each R¹¹ is independently selected from hydrogen and C₁₋₆ alkyl;
R² is selected from hydrogen, C₁₋₆ alkyl, C₂₋₆ alkenyl, and C₂₋₆ alkynyl; and
each R³ is independently selected from C₁₋₆ alkyl, C₂₋₆ alkenyl, C₂₋₆ alkynyl, -OH, -O(C₁₋₆ alkyl), -O(C₁₋₆ alkylene)-OH, -O(C₁₋₆ alkylene)-O(C₁₋₆ alkyl), -(C₁₋₆ alkylene)-OH, -(C₁₋₆ alkylene)-O(C₁₋₆ alkyl), -SH, -S(C₁₋₆ alkyl), -S(C₁₋₆ alkylene)-SH, -S(C₁₋₆ alkylene)-S(C₁₋₆ alkyl), -(C₁₋₆ alkylene)-SH, -(C₁₋₆ alkylene)-S(C₁₋₆ alkyl), -NH₂, -NH(C₁₋₆ alkyl), -N(C₁₋₆ alkyl)(C₁₋₆ alkyl), -NH-OH, -N(C₁₋₆ alkyl)-OH, -NH-O(C₁₋₆ alkyl), -N(C₁₋₆ alkyl)-O(C₁₋₆ alkyl), =NH, =N(C₁₋₆ alkyl), halogen, C₁₋₆ haloalkyl, -O-(C₁₋₆ haloalkyl), -CN, -NO₂, -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), -SO-(C₁₋₆ alkyl), -(C₀₋₄ alkylene)-carbocyclyl, and -(C₀₋₄ alkylene)-heterocyclyl, wherein the carbocyclyl group in said -(C₀₋₄ alkylene)-carbocyclyl and the heterocyclyl group in said -(C₀₋₄ alkylene)-heterocyclyl are each optionally substituted with one or more groups independently selected from C₁₋₆ alkyl, C₂₋₆ alkenyl, C₂₋₆ alkynyl, halogen, C₁₋₆ haloalkyl, -O-(C₁₋₆ haloalkyl), -CN, -OH, -O(C₁₋₆ alkyl), -SH, -S(C₁₋₆ alkyl), -NH₂, -NH(C₁₋₆ alkyl), -N(C₁₋₆ alkyl)(C₁₋₆ alkyl), -CHO, -CO(C₁₋₆ alkyl), -COOH, -COO(C₁₋₆ alkyl), -O-CO(C₁₋₆ alkyl), -CO-NH₂, -CO-NH(C₁₋₆ alkyl), -CO-N(C₁₋₆ alkyl)(C₁₋₆ alkyl), -NH-CO(C₁₋₆ alkyl), -N(C₁₋₆ alkyl)-CO(C₁₋₆ alkyl), -NH-COO(C₁₋₆ alkyl), -N(C₁₋₆ alkyl)-COO(C₁₋₆ alkyl), -O-CO-NH(C₁₋₆ alkyl), -O-CO-N(C₁₋₆ alkyl)(C₁₋₆ alkyl), -SO₂-NH₂, -SO₂-NH(C₁₋₆ alkyl), -SO₂-N(C₁₋₆ alkyl)(C₁₋₆ alkyl), -NH-SO₂-(C₁₋₆ alkyl), -N(C₁₋₆ alkyl)-SO₂-(C₁₋₆ alkyl), -SO₂-(C₁₋₆ alkyl), and -SO-(C₁₋₆ alkyl);
or a pharmaceutically acceptable salt thereof,
for use in therapy.

2. The compound for use according to claim 1, wherein R¹ is selected from hydrogen, C₁₋₈ alkyl, C₂₋₈ alkenyl, C₂₋₈ alkynyl, -(C₁₋₆ alkylene)-OH, -(C₁₋₆ alkylene)-O(C₁₋₆ alkyl), -(C₁₋₆ alkylene)-SH, -(C₁₋₆ alkylene)-S(C₁₋₆ alkyl), -(C₁₋₆ alkylene)-NH₂, -(C₁₋₆ alkylene)-NH-CO-NH₂, -(C₁₋₆ alkylene)-NH-C(=NH)-NH₂, -(C₁₋₆ alkylene)-O-CO-NH₂, -(C₁₋₆ alkylene)-COOH, -(C₁₋₆ alkylene)-CO-NH₂, -(C₁₋₆ alkylene)-CO-NH-OH, -(C₁₋₆ alkylene)-SO₃H, phenyl, -(C₁₋₆ alkylene)-phenyl, cycloalkyl, -(C₁₋₆ alkylene)-cycloalkyl, heteroaryl, -(C₁₋₆ alkylene)-heteroaryl, heterocycloalkyl, and -(C₁₋₆ alkylene)-heterocycloalkyl, wherein said phenyl, the phenyl group in said -(C₁₋₆ alkylene)-phenyl, said cycloalkyl, the cycloalkyl group in said -(C₁₋₆ alkylene)-cycloalkyl, said heteroaryl, the heteroaryl group in said -(C₁₋₆ alkylene)-heteroaryl, said heterocycloalkyl, and the heterocycloalkyl group in said -(C₁₋₆ alkylene)-heterocycloalkyl are each optionally substituted with one or more groups R³, and further wherein said alkyl, said alkenyl, said alkynyl, and any alkylene group in any of the aforementioned groups are each optionally substituted with one or more -OH.

3. The compound for use according to claim 1 or 2, wherein R¹ is selected from hydrogen, methyl, ethyl, n-propyl, isopropyl, n-butyl, isobutyl, sec-butyl, tert-butyl, n-pentyl, n-hexyl, -CH₂-CH=CH-CH₃, -CH₂-C≡CH, phenyl, 4-hydroxyphenyl, 3,5-dihydroxyphenyl, 3,5-dichloro-4-hydroxyphenyl, -CH₂-phenyl, -CH₂-(4-hydroxyphenyl), -CH₂-(3-hydroxyphenyl), -CH₂-(3,4-dihydroxyphenyl), -CH₂-(3-nitro-4-hydroxyphenyl), -CH₂-(4-methoxyphenyl), -CH₂-(3-hydroxy-4-methoxy-5-methylphenyl), -CH₂-(4-prenyloxyphenyl), -CH₂-(4-(N,N-dimethylamino)phenyl), -CH(-OH)-phenyl, -CH(-OH)-(4-hydroxyphenyl), - CH(-OH)-(4-methoxyphenyl), -CH(-OH)-(4-aminophenyl), -CH(-OH)-(4-nitrophenyl), -CH(-CH₃)-phenyl, -CH₂CH₂-phenyl, -CH₂CH₂-(4-hydroxyphenyl), -CH(-OH)-CH(-OH)-(4-hydroxyphenyl), -CH(-OH)-CH₂-(4-nitrophenyl), -CH₂-(1H-indol-3-yl), -CH(-CH₃)-(1H-indol-3-yl), -CH(-OH)-(1H-indol-3-yl), -CH₂-(5-hydroxy-1H-indol-3-yl), -CH₂-(1-acetyl-6-hydroxy-1H-indol-3-yl), -CH₂-(1-(1,1-dimethylallyl)-1H-indol-3-yl), - CH(-CH₃)-(2-oxo-indolin-3-yl), -CH₂-(4-nitro-1H-indol-3-yl), -CH₂-(5-nitro-1H-indol-3-yl), -CH₂-(5-chloro-1H-indol-3-yl), -CH₂-(6-chloro-1H-indol-3-yl), -CH₂-(6-bromo-1H-indol-3-yl), -CH₂-(7-fluoro-1H-indol-3-yl), -CH₂-(7-chloro-1H-indol-3-yl), -CH₂-(7-bromo-1H-indol-3-yl), -CH₂-(6,7-dichloro-1H-indol-3-yl), -CH₂-(1H-imidazol-4-yl), -CH(-OH)-(1H-imidazol-4-yl), -CH₂-(5-mercapto-1-methyl-1H-imidazol-4-yl), -CH₂-(3-furanyl), -CH₂-(3-pyridinyl), -CH(-CH₃)-CH(-OH)-(5-hydroxypyridin-2-yl), pyrrolidin-2-yl, -CH₂-(2-imino-4-imidazolidinyl), 2-iminohexahydropyrimidin-4-yl, 1-methylcycloprop-1-yl, -CH₂-(2-nitrocycloprop-1-yl), -CH₂-OH, -CH₂CH₂-OH, -CH₂-O-CH₃, -CH₂CH₂-O-CH₃, -CH₂CH₂-O-CH₂CH₃, -CH(-CH₃)-OH, -C(-CH₃)(-OH)-CH₃, -CH(-OH)-CH(-CH₃)-CH₃, -CH(-OH)-CH(-CH₃)-CH₂-CH=CH-CH₃, -CH(-OH)-C≡CH, -CH₂-SH, -CH₂CH₂-SH, -CH₂CH₂-S-CH₃, -CH₂CH₂-S-CH₂CH₃, -CH₂-SO₃H, -CH₂-COOH, -CH₂CH₂-COOH, -CH(-OH)-COOH, -CH(-OH)-CH₂-COOH, -CH(-CH₃)-COOH, -CH(-CH₃)-CH₂-COOH, -CH₂-CO-NH₂, -CH₂CH₂-CO-NH₂, -CH(-OH)-CO-NH₂, -CH₂-NH-CO-NH₂, -(CH₂)₂-NH-CO-NH₂, -(CH₂)₃-NH-CO-NH₂, -(CH₂)₂-CO-NH-OH, -CH₂-NH₂, -(CH₂)₂-NH₂, -(CH₂)₃-NH₂, -(CH₂)₄-NH₂, -CH(-CH₃)-NH₂, -CH(-CH₃)-(CH₂)₂-NH₂, -C(-OH)-(CH₂)₃-NH₂, -CH₂-C(-OH)-(CH₂)₂-NH₂, -(CH₂)₂-C(-OH)-C(-CH₂-OH)-NH₂, -(CH₂)₃-NH-C(=NH)-NH₂, -CH(-CH₃)-(CH₂)₂-NH-C(=NH)-NH₂, -C(-OH)-(CH₂)₂-NH-C(=NH)-NH₂, -CH₂-C(-OH)-CH₂-NH-C(=NH)-NH₂, -C(-OH)-C(-OH)-CH₂-NH-C(=NH)-NH₂, -(CH₂)₄-NH-C(=NH)-NH₂, -C(-OH)-(CH₂)₃-NH-C(=NH)-NH₂, -CH₂-C(-OH)-CH₂CH₂-NH-C(=NH)-NH₂, and -CH(-OH)-CH(-OH)-CH₂-O-CO-NH₂.

4. The compound for use according to any one of claims 1 to 3, wherein R² is hydrogen.

5. The compound for use according to any one of claims 1 to 4, wherein said compound has the following absolute configuration:

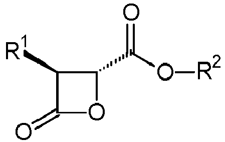

6. The compound for use according to claim 1, wherein said compound is any one of the following compounds or a pharmaceutically acceptable salt thereof: 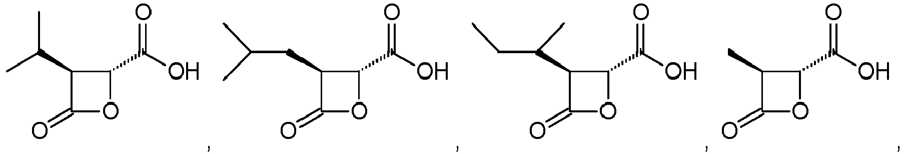 or

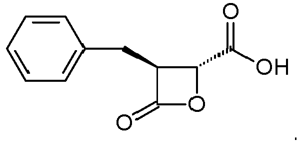

7. The compound for use according to claim 1, wherein said compound is 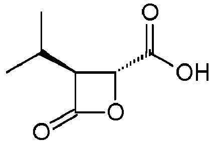 or a pharmaceutically acceptable salt thereof.

8. A pharmaceutical composition comprising a compound as defined in any one of claims 1 to 7 and a pharmaceutically acceptable excipient.

9. A compound as defined in any one of claims 1 to 7 or the pharmaceutical composition of claim 8 for use in the treatment or prevention of cancer, an infectious disease, an inflammatory disease, or an autoimmune disease.

10. The compound for use according to claim 9 or the pharmaceutical composition for use according to claim 9, for use in the treatment or prevention of cancer, wherein said cancer is selected from lung cancer, renal cancer, gastro-intestinal cancer, stomach cancer, colorectal cancer, colon cancer, malignant familial adenomatous polyposis, anal cancer, genitourinary cancer, bladder cancer, liver cancer, pancreatic cancer, ovarian cancer, cervical cancer, endometrial cancer, vaginal cancer, vulvar cancer, prostate cancer, testicular cancer, biliary tract cancer, hepatobiliary cancer, neuroblastoma, brain cancer, breast cancer, head and/or neck cancer, skin cancer, melanoma, Merkel-cell carcinoma, epidermoid cancer, squamous cell carcinoma, bone cancer, fibrosarcoma, Ewing's sarcoma, malignant mesothelioma, esophageal cancer, laryngeal cancer, mouth cancer, thymoma, neuroendocrine cancer, hematological cancer, leukemia, lymphoma, and multiple myeloma.

11. The compound for use according to claim 9 or the pharmaceutical composition for use according to claim 9, for use in the treatment or prevention of an infectious disease, wherein said infectious disease is selected from a bacterial infectious disease, a protozoan infectious disease, a fungal infectious disease, and a helminth infectious disease.

12. A plant protection composition comprising a compound of formula (I) as defined in any one of claims 1 to 7 or an agriculturally acceptable salt thereof.

13. A method of controlling a plant disease, the method comprising applying a compound of formula (I) as defined in any one of claims 1 to 7 or an agriculturally acceptable salt thereof or the plant protection composition of claim 12;
preferably wherein the plant disease is caused by a plant pathogenic organism selected from a bacterium, a protozoan, a fungus, an oomycete, a nematode, and an insect.

14. A disinfectant comprising a compound of formula (I) as defined in any one of claims 1 to 7 or a salt thereof.

15. *In vitro* use of a compound as defined in any one of claims 1 to 7 as a proteasome inhibitor.
